# EUROPÄISCHE PATENTSCHRIFT

(11) **EP 2 668 158 B1**
(45) Veröffentlichungstag und Bekanntmachung des Hinweises auf die Patenterteilung: **27.06.2018**
(21) Anmeldenummer: 12700832.4
(22) Anmeldetag: 20.01.2012
(51) Int. Cl.: C07D 207/36, C07D 491/113, C07D 491/107, C07C 255/46, C07D 317/72, C07C 235/74, C07D 209/54, C07D 307/22

(54) **VERFAHREN ZUR HERSTELLUNG VON 1-H-PYRROLIDIN-2,4-DION-DERIVATEN**
METHOD FOR PRODUCING 1-H-PYRROLIDINE-2,4-DIONE DERIVATIVES
PROCÉDÉ DE PRODUCTION DE DÉRIVÉS DE LA 1-H-PYRROLIDINE-2,4-DIONE

(30) Priorität: 25.01.2011 EP 11152069; 25.01.2011 US 201161435910 P
(43) Veröffentlichungstag der Anmeldung: 04.12.2013
(60) Teilanmeldung: 18162778.7
(73) Patentinhaber: Bayer Intellectual Property GmbH, 40789 Monheim am Rhein (DE)
(72) Erfinder: FISCHER, Reiner, 40789 Monheim (DE); HIMMLER, Thomas, 51519 Odenthal (DE)
(74) Vertreter: BIP Patents
(86) Internationale Anmeldenummer: PCT/EP2012/050840
(87) Internationale Veröffentlichungsnummer: WO 2012/101047

(56) Entgegenhaltungen:
- DE-A1- 4 409 044
- DE-A1- 19 515 690
- WAYLAND E. NOLAND, RICHARD J. SUNDBERG: "Structure of the 2:2 Condensation Product of Nitromethane and Cyclohexanone", JOURNAL OF ORGANIC CHEMISTRY, Bd. 28, Nr. 11, 1963, Seiten 3150-3165, XP002632565, DOI: 10.1021/jo01046a051
- HATSUHIKO MIZUNO,SHIRO TERASHIMA AND SHUNICHI YAMADA: "Stereochemical Studies. VII. Thermal Rearrangement of alpha-Hydroxyimines to alpha-Aminoketones using optically Active Open Chain Compounds", CHEMICAL & PHARMACEUTICAL BULLETIN, Bd. 19, Nr. 2, 1971, Seiten 227-246, XP002632566,
- SATOH, SHIGERU; ESASHI, YOHJI: "In vivo formation of 1-malonylaminocyclopropane-1-carboxylic acid and its relationship to ethylene production in cocklebur seed segments: a tracer study with 1-amino-2-ethylcyclopropane-1-carboxylic acid", PHYTOCHEMISTRY, Bd. 23, Nr. 8, 1984, Seiten 1561-1565, XP002632567, DOI: 10.1016/S0031-9422(00)83439-1
- MORTEN STORGAARD ET AL: "Palladium-Catalyzed r-Arylation of Tetramic Acids", JOURNAL OF ORGANIC CHEMISTRY, AMERICAN CHEMICAL SOCIETY, EASTON.; US, Bd. 74, Nr. 14, 27. Mai 2009 (2009-05-27), Seiten 5032-5040, XP007918205, ISSN: 0022-3263 [gefunden am 2009-05-27] in der Anmeldung erwähnt

## Beschreibung

Die vorliegende Erfindung betrifft ein neues Verfahren zur Herstellung von 1-H-Pyrrolidin-2,4-dion-Derivaten.

1-H-Pyrrolidin-2,4-dion-Derivate mit akarizider, insektizider, fungizider und herbizider Wirkung sind bekannt:
EP-A-456 063, EP-A-521 334, EP-A-596 298, EP-A-613 884, EP-A-613 885, WO 95/01 971, WO 95/26 954, WO 95/20 572, EP-A-0 668 267, WO 96/25 395, WO 96/35 664, WO 97/01 535, WO 97/02 243, WO 97/36 868, WO 97/43275, WO 98/05638, WO 98/06721, WO 98/25928, WO 99/24437, WO 99/43649, WO 99/48869, WO 99/55673, WO 01/17972, WO 01/23354, WO 01/74770, WO 03/013249, WO 03/062244, WO 2004/007448, WO 2004/024 688, WO 04/065366, WO 04/080962, WO 04/111042, WO 05/044791, WO 05/044796, WO 05/048710, WO 05/049569, WO 05/066125, WO 05/092897, WO 06/000355, WO 06/029799, WO 06/056281, WO 06/056282, WO 06/089633, WO 07/048545, DEA 102 00505 9892, WO 07/073856, WO 07/096058, WO 07/121868, WO 07/140881, WO 08/067873, WO 08/067910, WO 08/067911, WO 08/138551, WO 09/015801, WO 09/039975, WO 09/049851, WO 09/115262, WO 10/052161, WO 10/102758, WO 10/063378, WO 10/063670, WO 10/102758, WO 2011/098443, WO 2011/098440, WO 11/067135, WO 11/067240, EP-Anmeldenummer 11154805.3. Außerdem sind ketalsubstituierte 1-H-Arylpyrrolidin-2,4-dione aus WO 99/16748 bekannt. Mit pharmazeutischer Wirkung sind bekannt WO 2011/098433, DE-A-102010008642, DE-A-102010008643 und DE-Anmeldenummer 102010008640.

Auch sind biphenylsubstituierte 1H-Pyrrolidin-dion Derivate mit fungizider Wirkung bekannt (WO 03/059065).

Es handelt sich dabei um Verbindungen der Formel (I) 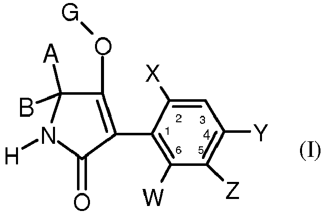 in welcher
- W: für Wasserstoff, Halogen, Alkyl, Alkenyl, Alkinyl, gegebenenfalls substituiertes Cycloalkyl, Alkoxy, Halogenalkyl oder Halogenalkoxy steht,
- X: für Wasserstoff, Halogen, Alkyl, Alkenyl, Alkinyl, gegebenenfalls substituiertes Cycloalkyl, Alkoxy, Halogenalkyl, Halogenalkoxy oder Cyano steht,
- Y und Z: unabhängig voneinander für Wasserstoff, Alkyl, Alkenyl, Alkinyl, Halogen, Cyano, gegebenenfalls substituiertes Cycloalkyl, Alkoxy, Halogenalkyl, Halogenalkoxy oder jeweils gegebenenfalls substituiertes Aryl oder Hetaryl stehen,
- A: für Wasserstoff, für jeweils gegebenenfalls durch Halogen substituiertes Alkyl, Alkenyl, Alkoxyalkyl, Alkylthioalkyl, gesättigtes oder ungesättigtes, gegebenenfalls substituiertes Cycloalkyl, in welchem gegebenenfalls mindestens ein Ringatom durch ein Heteroatom ersetzt ist, oder jeweils gegebenenfalls durch Halogen, Alkyl, Halogenalkyl, Alkoxy, Halogenalkoxy, Cyano oder Nitro substituiertes Aryl, Arylalkyl oder Hetaryl steht,
- B: für Wasserstoff, Alkyl oder Alkoxyalkyl steht, oder
- A und B: gemeinsam mit dem Kohlenstoffatom an das sie gebunden sind für einen gesättigten oder ungesättigten, gegebenenfalls mindestens ein Heteroatom enthaltenden unsubstituierten oder substituierten Cyclus stehen,
- G: für Wasserstoff (a) oder für eine der Gruppen 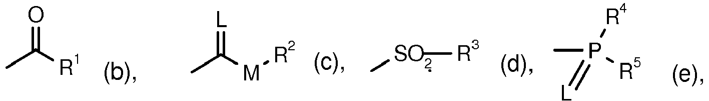 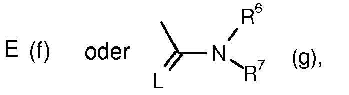
steht,
worin
- E: für ein Metallion oder ein Ammoniumion steht,
- L: für Sauerstoff oder Schwefel steht,
- M: für Sauerstoff oder Schwefel steht,
- R¹: für jeweils gegebenenfalls durch Halogen oder Cyano substituiertes Alkyl, Alkenyl, Alkoxy-alkyl, Alkylthioalkyl oder Polyalkoxyalkyl oder für jeweils gegebenenfalls durch Halogen, Alkyl oder Alkoxy substituiertes Cycloalkyl oder Heterocyclyl oder für jeweils gegebenenfalls substituiertes Phenyl, Phenylalkyl, Hetaryl, Phenoxyalkyl oder Hetaryloxyalkyl steht,
- R²: für jeweils gegebenenfalls durch Halogen oder Cyano substituiertes Alkyl, Alkenyl, Alkoxy-alkyl oder Polyalkoxyalkyl oder für jeweils gegebenenfalls substituiertes Cycloalkyl, Phenyl oder Benzyl steht,
- R³, R⁴ und R⁵: unabhängig voneinander für jeweils gegebenenfalls durch Halogen substituiertes Alkyl, Alkoxy, Alkylamino, Dialkylamino, Alkylthio, Alkenylthio oder Cycloalkylthio oder für jeweils gegebenenfalls substituiertes Phenyl, Benzyl, Phenoxy oder Phenylthio stehen,
- R⁶ und R⁷: unabhängig voneinander für Wasserstoff, für jeweils gegebenenfalls durch Halogen oder Cyano substituiertes Alkyl, Cycloalkyl, Alkenyl, Alkoxy, Alkoxyalkyl, für jeweils gegebenenfalls substituiertes Phenyl oder Benzyl stehen, oder gemeinsam mit dem N-Atom, an das sie gebunden sind, einen gegebenenfalls Sauerstoff oder Schwefel enthaltenden und gegebenenfalls substituierten Cyclus bilden.

Die Verbindungen der Formel (I) können, auch in Abhängigkeit von der Art der Substituenten, als optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Unter Einbeziehung der verschiedenen Bedeutungen (a), (b), (c), (d), (e), (f) und (g) der Gruppe G ergeben sich folgende hauptsächliche Strukturen (I-a) bis (I-g), 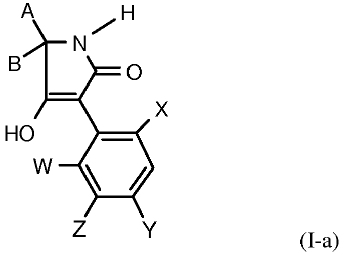 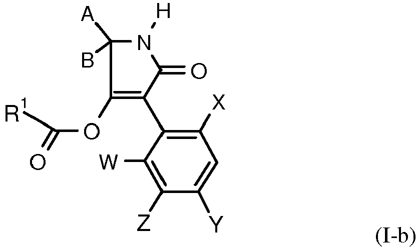 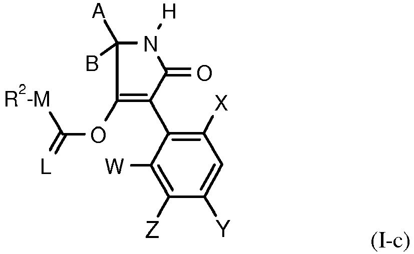 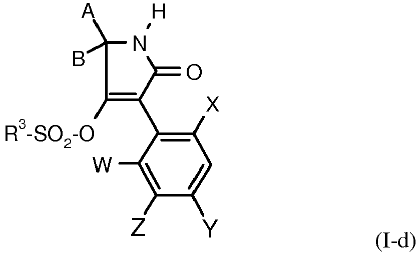 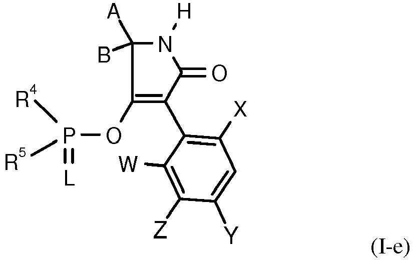 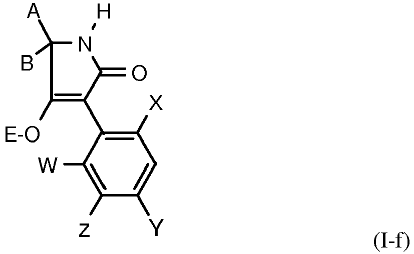 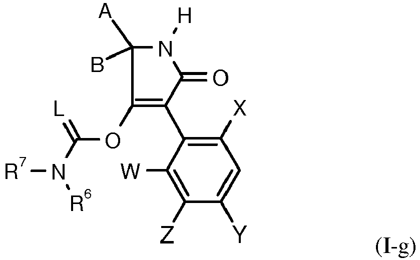 worin
A, B, E, L, M, W, X, Y, Z, R¹, R², R³, R⁴, R⁵, R⁶ und R⁷ die oben angegebenen Bedeutungen besitzen.

Weiterhin ist bereits bekannt, dass man die Verbindungen der Formel (I) nach den im Folgenden beschriebenen Verfahren erhält:
(A*) Man erhält Verbindungen der Formel (I-a) 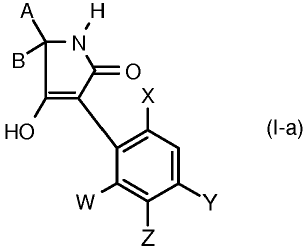 in welcher
   A, B, W, X, Y und Z die oben angegebenen Bedeutungen haben,
   wenn man
   Verbindungen der Formel (II) 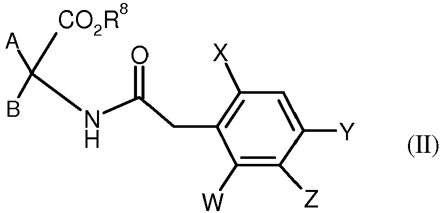 in welcher
   A, B, W, X, Y und Z die oben angegebenen Bedeutungen haben,
      und
   R⁸ für Alkyl steht,
   in Gegenwart eines Verdünnungsmittels und in Gegenwart einer Base intramolekular kondensiert.
   Außerdem ist bekannt
(B*) dass man die Verbindungen der oben gezeigten Formeln (I-b), in welchen R¹, A, B, W, X, Y und Z die oben angegebenen Bedeutungen haben, erhält, wenn man Verbindungen der oben gezeigten Formeln (I-a), in welchen A, B, W, X, Y und Z die oben angegebenen Bedeutungen haben, jeweils
α) mit Verbindungen der Formel (III) 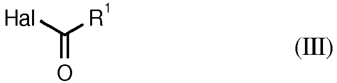 in welcher
   - R¹: die oben angegebene Bedeutung hat und
   - Hal: für Halogen (insbesondere Chlor oder Brom) steht
   oder
β) mit Carbonsäureanhydriden der Formel (IV)

   R¹-CO-O-CO-R¹ (IV)

   in welcher
   - R¹: die oben angegebene Bedeutung hat,
   gegebenenfalls in Gegenwart eines Verdünnungsmittels und gegebenenfalls in Gegenwart eines Säurebindemittels umsetzt;
(C*) dass man die Verbindungen der oben gezeigten Formeln (I-c), in welchen R², A, B, M, W, X, Y und Z die oben angegebenen Bedeutungen haben und L für Sauerstoff steht, erhält, wenn man Verbindungen der oben gezeigten Formeln (I-a), in welchen A, B, W, X, Y und Z die oben angegebenen Bedeutungen haben, jeweils
   mit Chlorameisensäureestern oder Chlorameisensäurethioestern der Formel (V)

   R²-M-CO-Cl (V)

   in welcher
   R² und M die oben angegebenen Bedeutungen haben,
   gegebenenfalls in Gegenwart eines Verdünnungsmittels und gegebenenfalls in Gegenwart eines Säurebindemittels umsetzt;
(D*) dass man Verbindungen der oben gezeigten Formeln (I-f), in welchen E, A, B, W, X, Y und Z die oben angegebenen Bedeutungen haben, erhält, wenn man Verbindungen der Formeln (I-a), in welchen A, B, W, X, Y und Z die oben angegebenen Bedeutungen haben, jeweils
   mit Metallverbindungen oder Aminen der Formeln (XIII) oder (XIV) 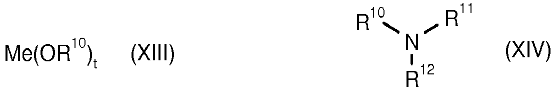 in welchen
   Me für ein ein- oder zweiwertiges Metall (bevorzugt ein Alkali- oder Erdalkalimetall wie Lithium, Natrium, Kalium, Magnesium oder Calcium),
   t für die Zahl 1 oder 2 und
   R¹⁰, R¹¹, R¹² unabhängig voneinander für Wasserstoff oder Alkyl (bevorzugt C₁-C₈-Alkyl) stehen,
   gegebenenfalls in Gegenwart eines Verdünnungsmittels umsetzt.

Die Verbindungen sind durch die Formel (I) allgemein definiert. Bevorzugte Substituenten bzw. Bereiche der in der oben und nachstehend erwähnten Formeln aufgeführten Reste werden im Folgenden erläutert:
- W: steht bevorzugt für Wasserstoff, Chlor, Brom, C₁-C₄-Alkyl, C₂-C₄-Alkenyl, C₂-C₄-Alkinyl, gegebenenfalls einfach durch Methyl, Ethyl, Methoxy, Fluor, Chlor, Trifluormethyl oder Cyclopropyl substituiertes C₃-C₆-Cycloalkyl, C₁-C₄-Alkoxy, C₁-C₂-Halogenalkyl oder C₁-C₂-Halogenalkoxy,
- X: steht bevorzugt für Wasserstoff, Chlor, Brom, Iod, C₁-C₄-Alkyl, C₂-C₄-Alkenyl, C₂-C₄-Alkinyl, gegebenenfalls einfach durch Methyl, Ethyl, Methoxy, Fluor, Chlor, Trifluormethyl oder Cyclopropyl substituiertes C₃-C₆-Cycloalkyl, C₁-C₄-Alkoxy, C₁-C₄-Halogenalkyl, C₁-C₄-Halogenalkoxy oder Cyano,
- Y und Z: stehen bevorzugt unabhängig voneinander für Wasserstoff, Cyano, Fluor, Chlor, Brom, Iod, C₁-C₄-Alkyl, C₂-C₄-Alkenyl, C₂-C₄-Alkinyl, gegebenenfalls einfach durch Methyl, Ethyl, Methoxy, Fluor, Chlor, Trifluormethyl oder Cyclopropyl substituiertes C₃-C₆-Cycloalkyl, C₁-C₆-Alkoxy, C₁-C₄-Halogenalkyl, C₁-C₄-Halogenalkoxy, oder für einen der (Het)-arylreste, 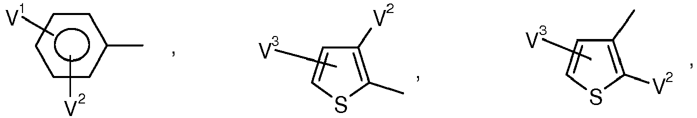 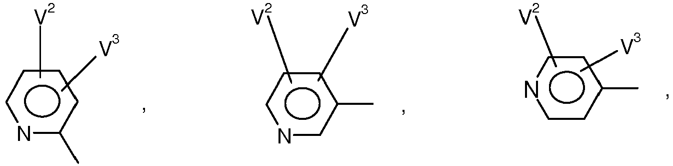 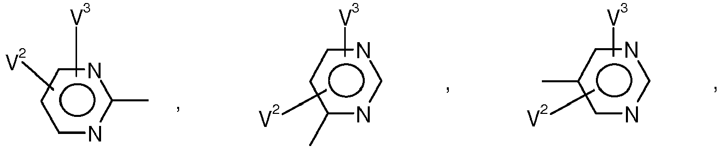
wobei im Falle von (Het)-aryl nur einer der Reste Y oder Z für (Het)-aryl stehen darf,
- V¹: steht bevorzugt für Wasserstoff, Fluor, Chlor, Brom, C₁-C₆-Alkyl, C₁-C₄-Alkoxy, C₁-C₂-Halogenalkyl, C₁-C₂-Halogenalkoxy, Nitro, Cyano oder gegebenenfalls einfach bis zweifach durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenyl,
- V² und V³: stehen bevorzugt unabhängig voneinander für Wasserstoff, Fluor, Chlor, Brom, C₁-C₄-Alkyl, C₁-C₄-Alkoxy, C₁-C₂-Halogenalkyl oder C₁-C₂-Halogenalkoxy,
- A: steht bevorzugt für Wasserstoff, jeweils gegebenenfalls einfach bis dreifach durch Fluor oder Chlor substituiertes C₁-C₆-Alkyl, C₁-C₄-Alkoxy-C₁-C₂-alkyl, gegebenenfalls einfach bis zweifach durch C₁-C₂-Alkyl oder C₁-C₂-Alkoxy substituiertes und gegebenenfalls durch ein Sauerstoffatom unterbrochenes C₃-C₆-Cycloalkyl oder jeweils gegebenenfalls einfach bis zweifach durch Fluor, Chlor, Brom, C₁-C₄-Alkyl, C₁-C₂-Halogenalkyl, C₁-C₄-Alkoxy, C₁-C₂-Halogenalkoxy, Cyano oder Nitro substituiertes Phenyl, Pyridyl oder Benzyl,
- B: steht bevorzugt für Wasserstoff, C₁-C₄-Alkyl oder C₁-C₂-Alkoxyl-C₁-C₂-alkyl oder
- A, B: und das Kohlenstoffatom an das sie gebunden sind, stehen bevorzugt für gesättigtes oder ungesättigtes C₃-C₇-Cycloalkyl, worin gegebenenfalls ein Ringglied durch Stickstoff, Sauerstoff oder Schwefel ersetzt ist und welches gegebenenfalls einfach bis zweifach durch Halogen, C₁-C₆-Alkyl, C₁-C₄-Alkoxy-C₁-C₂-alkyl, Trifluormethyl, C₁-C₆-Alkoxy, C₃-C₆-Alkenyloxy, Trifluorethoxy, C₁-C₃-Alkoxy-C₁-C₃-alkoxy oder C₃-C₆-Cycloalkylmethoxy substituiert ist, wobei die zuvor genannten Reste (ausgenommen Halogen und Trifluormethyl) auch als N-Substituenten in Frage kommen, oder
- A, B: und das Kohlenstoffatom, an das sie gebunden sind, stehen bevorzugt für C₅-C₆-Cycloalkyl, welches durch eine gegebenenfalls mit ein oder zwei nicht direkt benachbarte Sauerstoff- oder Schwefelatome enthaltende gegebenenfalls durch Methyl oder Ethyl substituierte Alkylendiyl- oder durch eine Alkylendioxyl- oder durch eine Alkylendithiol-Gruppe substituiert ist, die mit dem Kohlenstoffatom, an das sie gebunden ist, einen weiteren fünf- oder sechsgliedrigen Ring bildet, oder
- A, B: und das Kohlenstoffatom, an das sie gebunden sind, stehen bevorzugt für C₃-C₆-Cycloalkyl oder C₅-C₆-Cycloalkenyl, in welchen zwei Substituenten gemeinsam mit den Kohlenstoffatomen, an die sie gebunden sind, für jeweils gegebenenfalls durch C₁-C₂-Alkyl oder C₁-C₂-Alkoxy substituiertes C₂-C₄-Alkandiyl, C₂-C₄-Alkendiyl oder Butadiendiyl stehen,
- G: steht bevorzugt für Wasserstoff (a) oder für eine der Gruppen 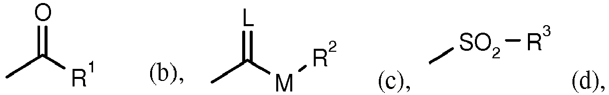 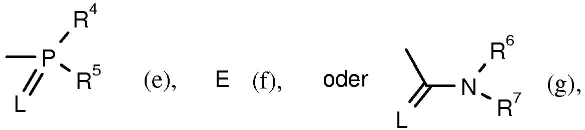
in welchen
- E: für ein Metallion oder ein Ammoniumion steht,
- L: für Sauerstoff oder Schwefel steht und
- M: für Sauerstoff oder Schwefel steht,
- R¹: steht bevorzugt für jeweils gegebenenfalls einfach bis dreifach durch Fluor oder Chlor substituiertes C₁-C₁₆-Alkyl, C₂-C₁₆-Alkenyl, C₁-C₆-Alkoxy-C₁-C₄-alkyl, C₁-C₆-Alkylthio-C₁-C₄-alkyl oder Poly-C₁-C₆-alkoxy-C₁-C₄-alkyl oder für gegebenenfalls einfach bis zweifach durch Fluor, Chlor, C₁-C₅-Alkyl oder C₁-C₅-Alkoxy substituiertes C₃-C₇-Cycloalkyl, in welchem gegebenenfalls eine oder zwei nicht direkt benachbarte Methylengruppen durch Sauerstoff und/oder Schwefel ersetzt sind,
für gegebenenfalls einfach bis dreifach durch Fluor, Chlor, Brom, Cyano, Nitro, C₁-C₄-Alkyl, C₁-C₄-Alkoxy, C₁-C₃-Halogenalkyl, C₁-C₃-Halogenalkoxy, C₁-C₄-Alkylthio oder C₁-C₄-Alkylsulfonyl substituiertes Phenyl,
für gegebenenfalls einfach bis zweifach durch Fluor, Chlor, Brom, C₁-C₄-Alkyl, C₁-C₄-Alkoxy, C₁-C₃-Halogenalkyl oder C₁-C₃-Halogenalkoxy substituiertes Phenyl-C₁-C₄-alkyl,
für jeweils gegebenenfalls einfach bis zweifach durch Fluor, Chlor, Brom oder C₁-C₄-Alkyl substituiertes Pyrazolyl, Thiazolyl, Pyridyl, Pyrimidyl, Furanyl oder Thienyl,
für gegebenenfalls einfach bis zweifach durch Fluor, Chlor, Brom oder C₁-C₄-Alkyl substituiertes Phenoxy-C₁-C₅-alkyl oder
für jeweils gegebenenfalls einfach bis zweifach durch Fluor, Chlor, Brom, Amino oder C₁-C₄-Alkyl substituiertes Pyridyloxy-C₁-C₅-alkyl, Pyrimidyloxy-C₁-C₅-alkyl oder Thiazolyloxy-C₁-C₅-alkyl,
- R²: steht bevorzugt für jeweils gegebenenfalls einfach bis dreifach durch Fluor oder Chlor substituiertes C₁-C₁₆-Alkyl, C₂-C₁₆-Alkenyl, C₁-C₆-Alkoxy-C₂-C₆-alkyl oder Poly-C₁-C₆-alkoxy-C₂-C₆-alkyl,
für gegebenenfalls einfach bis zweifach durch Fluor, Chlor, C₁-C₄-Alkyl oder C₁-C₄-Alkoxy substituiertes C₃-C₇-Cycloalkyl oder
für jeweils gegebenenfalls einfach bis dreifach durch Fluor, Chlor, Brom, Cyano, Nitro, C₁-C₄-Alkyl, C₁-C₃-Alkoxy, C₁-C₃-Halogenalkyl oder C₁-C₃-Halogenalkoxy substituiertes Phenyl oder Benzyl,
- R³: steht bevorzugt für gegebenenfalls einfach bis dreifach durch Fluor oder Chlor substituiertes C₁-C₆-Alkyl oder jeweils gegebenenfalls einfach bis zweifach durch Fluor, Chlor, Brom, C₁-C₄-Alkyl, C₁-C₄-Alkoxy, C₁-C₂-Halogenalkoxy, C₁-C₂-Halogenalkyl, Cyano oder Nitro substituiertes Phenyl oder Benzyl,
- R⁴ und R⁵: stehen unabhängig voneinander bevorzugt für jeweils gegebenenfalls einfach bis dreifach durch Fluor oder Chlor substituiertes C₁-C₆-Alkyl, C₁-C₆-Alkoxy, C₁-C₆-Alkylamino, Di-(C₁-C₆-alkyl)amino, C₁-C₆-Alkylthio oder C₃-C₄-Alkenylthio oder für jeweils gegebenenfalls einfach bis zweifach durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl, oder C₁-C₃-Halogenalkyl substituiertes Phenyl, Phenoxy oder Phenylthio,
- R⁶ und R⁷: stehen unabhängig voneinander bevorzugt für Wasserstoff, für jeweils gegebenenfalls einfach bis dreifach durch Fluor oder Chlor substituiertes C₁-C₆-Alkyl, C₃-C₆-Cycloalkyl, C₁-C₆-Alkoxy, C₃-C₆-Alkenyl oder C₁-C₆-Alkoxy-C₂-C₆-alkyl, für jeweils gegebenenfalls einfach bis dreifach durch Fluor, Chlor, Brom, C₁-C₅-Halogenalkyl, C₁-C₅-Alkyl oder C₁-C₅-Alkoxy substituiertes Phenyl oder Benzyl, oder zusammen für einen gegebenenfalls durch C₁-C₄-Alkyl substituierten C₃-C₆-Alkylenrest, in welchem gegebenenfalls eine Methylengruppe durch Sauerstoff oder Schwefel ersetzt ist.

In den als bevorzugt genannten Restedefinitionen steht Halogen für Fluor, Chlor und Brom, insbesondere für Fluor und Chlor.
- W: steht besonders bevorzugt für Wasserstoff, Chlor, Brom, Methyl, Ethyl, Methoxy, Ethoxy oder Trifluormethyl,
- X: steht besonders bevorzugt für Wasserstoff, Chlor, Brom, Jod, Methyl, Ethyl, Propyl, Cyclopropyl, Methoxy, Ethoxy, Trifluormethyl oder Trifluormethoxy,
- Y und Z: stehen besonders bevorzugt unabhängig voneinander für Wasserstoff, Fluor, Chlor, Brom, Iod, Methyl, Ethyl, Cyclopropyl, Methoxy, Trifluormethyl, Trifluormethoxy oder einen Phenylrest, 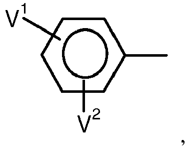
wobei im Falle von Phenyl nur einer der Reste Y oder Z für Phenyl stehen darf,
- V¹: steht besonders bevorzugt für Wasserstoff, Fluor oder Chlor,
- V²: steht besonders bevorzugt für Wasserstoff, Fluor, Chlor, Methyl, Ethyl, n-Propyl, iso-Propyl, Methoxy, Ethoxy oder Trifluormethyl,
- A: steht besonders bevorzugt für Wasserstoff, jeweils gegebenenfalls einfach bis dreifach durch Fluor substituiertes C₁-C₄-Alkyl oder C₁-C₂-Alkoxy-C₁-C₂-alkyl, für Cyclopropyl, Cyclopentyl oder Cyclohexyl,
- B: steht besonders bevorzugt für Wasserstoff, Methyl oder Ethyl oder
- A, B: und das Kohlenstoffatom an das sie gebunden sind, stehen besonders bevorzugt für gesättigtes C₅-C₆-Cycloalkyl, in welchem gegebenenfalls ein Ringglied durch Stickstoff, Sauerstoff oder Schwefel ersetzt ist und welches gegebenenfalls einfach oder zweifach durch Fluor, Chlor, Methyl, Ethyl, Methoxymethyl, Ethoxymethyl, Methoxyethyl, Ethoxyethyl, Trifluormethyl, Methoxy, Ethoxy, Propoxy, Butoxy, Methoxyethoxy, Ethoxyethoxy, Allyloxy, Trifluorethoxy oder Cyclopropylmethoxy substituiert ist, wobei die zuvor genannten Reste (ausgenommen Fluor, Chlor, Trifluormethyl) auch als N-Substituenten in Frage kommen,
- A, B: und das Kohlenstoffatom, an das sie gebunden sind, stehen besonders bevorzugt für C₆-Cycloalkyl, welches gegebenenfalls durch eine gegebenenfalls durch eine mit einem Sauerstoffatom unterbrochene Alkylidendiyl-Gruppe oder durch eine mit zwei nicht direkt benachbarten Sauerstoffatomen enthaltende Alkylidendiyl-Gruppe substituiert ist, wobei ein 5- oder 6-Ringketal gebildet wird und die jeweils gegebenenfalls einfach oder zweifach durch Methyl subsitutiert sein können, oder
- A, B: und das Kohlenstoffatom, an das sie gebunden sind, stehen besonders bevorzugt für C₅-C₆-Cycloalkyl oder C₅-C₆-Cycloalkenyl, worin zwei Substituenten gemeinsam mit den Kohlenstoffatomen, an die sie gebunden sind, für C₂-C₄-Alkandiyl oder C₂-C₄-Alkendiyl oder Butadiendiyl stehen,
- G: steht besonders bevorzugt für Wasserstoff (a) oder für eine der Gruppen 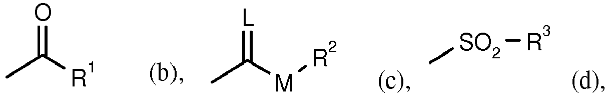 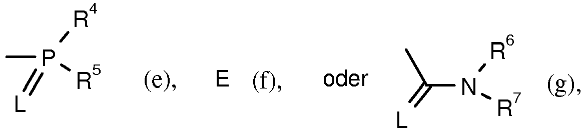
in welchen
- E: für ein Metallion oder ein Ammoniumion steht,
- L: für Sauerstoff oder Schwefel steht und
- M: für Sauerstoff oder Schwefel steht,
- R¹: steht besonders bevorzugt für jeweils gegebenenfalls einfach bis dreifach durch Fluor oder Chlor substituiertes C₁-C₁₀-Alkyl, C₂-C₁₀-Alkenyl, C₁-C₄-Alkoxy-C₁-C₂-alkyl, C₁-C₄-Alkylthio-C₁-C₂-alkyl oder für gegebenenfalls einfach durch Fluor, Chlor, Methyl, Ethyl oder Methoxy substituiertes C₃-C₆-Cycloalkyl, für gegebenenfalls einfach bis zweifach durch Fluor, Chlor, Brom, Cyano, Nitro, Methyl, Ethyl, n-Propyl, i-Propyl, Methoxy, Ethoxy, Trifluormethyl oder Trifluormethoxy substituiertes Phenyl, für jeweils gegebenenfalls einfach durch Chlor, Brom oder Methyl substituiertes Furanyl, Thienyl oder Pyridyl,
- R²: steht besonders bevorzugt für jeweils gegebenenfalls einfach bis dreifach durch Fluor oder Chlor substituiertes C₁-C₁₀-Alkyl, C₂-C₁₀-Alkenyl oder C₁-C₄-Alkoxy-C₂-C₄-alkyl, für Cyclopentyl oder Cyclohexyl oder für jeweils gegebenenfalls einfach bis zweifach durch Fluor, Chlor, Cyano, Nitro, Methyl, Ethyl, Methoxy, Trifluormethyl oder Trifluormethoxy substituiertes Phenyl oder Benzyl,
- R³: steht besonders bevorzugt für jeweils gegebenenfalls einfach bis dreifach durch Fluor oder Chlor substituiertes Methyl, Ethyl, Propyl oder iso-Propyl, oder jeweils gegebenenfalls einfach durch Fluor, Chlor, Brom, Methyl, Ethyl, iso-Propyl, tert.-Butyl, Methoxy, Ethoxy, iso-Propoxy, Trifluormethyl, Trifluormethoxy, Cyano oder Nitro substituiertes Phenyl,
- R⁴ und R⁵: stehen unabhängig voneinander besonders bevorzugt für C₁-C₄-Alkoxy oder C₁-C₄-Alkylthio oder für jeweils gegebenenfalls einfach durch Fluor, Chlor, Brom, Nitro, Cyano, Methyl, Methoxy, Trifluormethyl oder Trifluormethoxy substituiertes Phenyl, Phenoxy oder Phenylthio,
- R⁶ und R⁷: stehen unabhängig voneinander besonders bevorzugt für Wasserstoff, für C₁-C₄-Alkyl, C₃-C₆-Cycloalkyl, C₁-C₄-Alkoxy, C₃-C₄-Alkenyl oder C₁-C₄-Alkoxy-C₂-C₄-alkyl, für gegebenenfalls einfach bis zweifach durch Fluor, Chlor, Brom, Methyl, Methoxy oder Trifluormethyl substituiertes Phenyl, oder zusammen für einen C₅-C₆-Alkylenrest, in welchem gegebenenfalls eine Methylengruppe durch Sauerstoff oder Schwefel ersetzt ist.
- W: steht ganz besonders bevorzugt für Wasserstoff, Chlor, Methyl, Ethyl oder Methoxy, (hervorgehoben für Wasserstoff, Chlor oder Methyl),
- X: steht ganz besonders bevorzugt für Wasserstoff, Chlor, Methyl, Ethyl, Methoxy oder Ethoxy, (hervorgehoben für Wasserstoff, Chlor oder Methyl),
- Y und Z: stehen ganz besonders bevorzugt unabhängig voneinander für Wasserstoff, Chlor, Methyl oder für den Rest 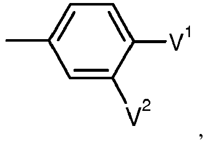 wobei in diesem Falle nur einer der Reste Y oder Z für 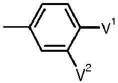 stehen darf, (hervorgehoben steht Z für 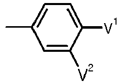 und Y für Wasserstoff)

- V¹: steht ganz besonders bevorzugt für Wassersoff, Fluor oder Chlor, (hervorgehoben für Wasserstoff oder Chlor),
- V²: steht ganz besonders bevorzugt für Wasserstoff, Fluor oder Chlor, (hervorgehoben für Wasserstoff),
- A: steht ganz besonders bevorzugt für Methyl, Ethyl, Propyl, iso-Propyl oder Cyclopropyl, (hervorgehoben für Methyl),
- B: steht ganz besonders bevorzugt für Wasserstoff oder Methyl, (hervorgehoben für Methyl),
- A, B: und das Kohlenstoffatom an das sie gebunden sind, stehen ganz besonders bevorzugt für gesättigtes C₅-C₆-Cycloalkyl, in welchem gegebenenfalls ein Ringglied durch Sauerstoff ersetzt ist und welches gegebenenfalls einfach durch Fluor, Chlor, Methyl, Ethyl, Methoxymethyl, Methoxy, Ethoxy, Propoxy, Butoxy, Trifluorethoxy substituiert ist, (hervorgehoben für C₆-Cycloalkyl, welches durch Methoxy substituiert ist), oder
- A, B: und das Kohlenstoffatom, an das sie gebunden sind, stehen ganz besonders bevorzugt für C₆-Cycloalkyl, welches gegebenenfalls durch eine gegebenenfalls durch eine mit Sauerstoff unterbrochene Alkylidendiyl-Gruppe oder durch eine mit zwei nicht direkt benachbarten
Sauerstoffatomen enthaltende Alkylidendiyl-Gruppe substituiert ist, wobei ein 5- oder 6-Ringketal gebildet wird, die jeweils einfach oder zweifach durch Methyl substituiert sein können,
- G: steht ganz besonders bevorzugt für Wasserstoff (a) oder für eine der Gruppen 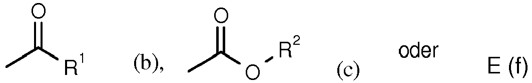
in welchen
- E: für ein Lithium-, Natrium-, Kalium-, Rubidium-, Cäsium-, Magnesium-, Calciumion oder ein Ammoniumion steht,
- R¹: steht ganz besonders bevorzugt für Methyl, Ethyl, Propyl, iso-Propyl, Butyl, iso-Butyl, sek.-Butyl, tert.-Butyl, Cyclopropyl, Cyclopentyl oder Cyclohexyl,
- R²: steht ganz besonders bevorzugt für Methyl, Ethyl, Propyl, iso-Propyl, Butyl, iso-Butyl, sek.-Butyl, tert.-Butyl, oder für Benzyl.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Die beim Verfahren (A*) als Ausgangsstoffe benötigten Verbindungen der Formel (II) 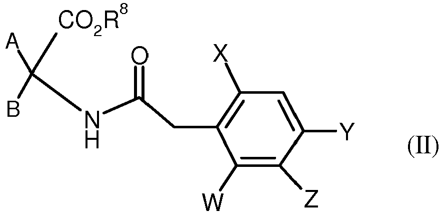 in welcher
A, B, W, X, Y, Z und R⁸ die oben angegebenen Bedeutungen haben,
erhält man beispielsweise, wenn man Aminosäurederivate der Formel (VI) 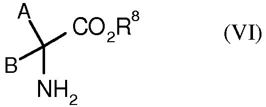 in welcher
A, B und R⁸ die oben angegebene Bedeutung haben,
mit substituierten Phenylessigsäurederivaten der Formel (VII) 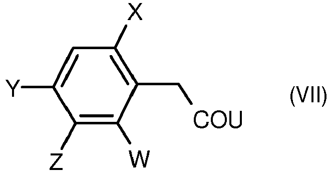 in welcher
W, X, Y und Z die oben angegebenen Bedeutungen haben und
U für eine durch Carbonsäureaktivierungsreagenzien wie Carbonyldiimidazol, Carbonyldiimide (wie z.B. Dicyclohexylcarbondiimid), Phosphorylierungsreagenzien (wie z.B. POCl₃, BOP-Cl), Halogenierungsmittel wie z.B. Thionylchlorid, Oxalylchlorid oder Phosgen sowie durch Benzolsulfonylchlorid oder Chlorameisensäureester eingeführte Abgangsgruppe steht,
acyliert (Chem. Reviews 52, 237-416 (1953); Bhattacharya, Indian J. Chem. 6, 341-5, 1968)
oder wenn man Acylaminosäuren der Formel (VIII) 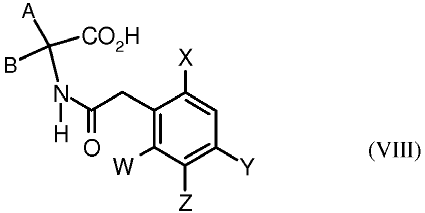 in welcher
A, B, W, X, Y und Z die oben angegebenen Bedeutungen haben,
verestert (Chem. Ind. (London) 1568 (1968)).

Man erhält die Verbindungen der Formel (VIII) beispielsweise, wenn man Aminocarbonsäuren der Formel (IX) 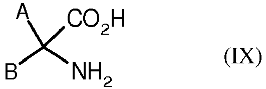 in welcher
A und B die oben angegebenen Bedeutungen haben
mit substituierten Phenylessigsäurederivaten der Formel (VII) 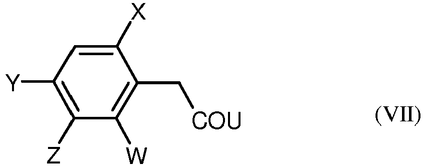 in welcher
U, W, X, Y und Z die oben angegebenen Bedeutungen haben
z.B. nach Schotten-Baumann acyliert (Organikum, VEB Deutscher Verlag der Wissenschaften, Berlin 1977, S. 505).

Die Verbindungen der Formeln (VI) und (IX) sind bekannt und lassen sich nach bekannten Verfahren darstellen (siehe z.B. Compagnon, Ann. Chim. (Paris) [14] 5, S. 11-22, 23-27 (1970), L. Munday, J. Chem. Soc. 4372 (1961); J.T. Eward, C. Jitrangeri, Can. J. Chem. 53, 3339 (1975), WO 02/02532 sowie wie in den eingangs zitierten Offenlegungsschriften beschrieben.

Für diese bekannten Verfahren werden also in jedem Fall substituierte Phenylessigsäurederivate der Formel (VII) 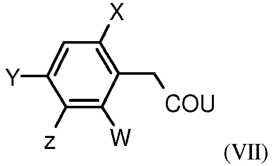 in welcher U, W, X, Y und Z die oben angegebenen Bedeutungen haben, benötigt.

Die Verbindungen der Formel (VII) sind aus den eingangs zitierten Offenlegungsschriften wie z. Bsp. WO 98/05638, WO 01/74770 bekannt oder lassen sich nach den dort beschriebenen Verfahren herstellen. Diese Verfahren sind teilweise technisch sehr aufwendig, vielstufig oder mit geringen Totalausbeuten belastet.

Es bestand daher weiterhin Bedarf an neuen Verfahren zur Herstellung von Verbindungen der allgemeinen Formel (I) 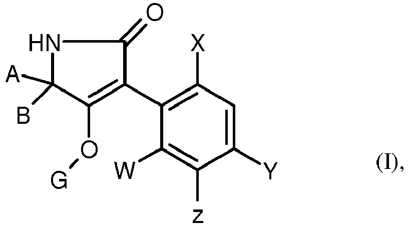 in welcher
A, B, W, X, Y, Z und G die oben angegebenen Bedeutungen haben, wobei X zusätzlich auch für Wasserstoff stehen darf, unter Vermeidung der Verwendung von Phenylessigsäurederivaten der Formel (VII).

Es ist bereits bekannt geworden, dass sich beispielsweise 1,3-Diketoverbindungen palladiumkatalysiert arylieren lassen (J.Amer.Chem.Soc. 2000, 122, 1360-70). Weiterhin wurde bekannt, dass sich Boc-geschützte Tetramsäuren palladiumkatalysiert ausgehend von Arylchloriden, -bromiden und -triflaten prinzipiell arylieren lassen, wobei die beschriebene Methode jedoch bei ortho-Substituenten versagt (J. Org. Chem. 2009, 74, 5032-5040). Da in keinem Beispiel der zitierten Literatur ein Substrat mit einer funktionellen NH-Gruppe, wie sie in Tetramsäuren der Formel (I) vorliegt, eingesetzt wurde, war im Gegenteil zu vermuten, dass solche Substrate dieser Reaktion nicht zugänglich seien, erst recht nicht mit orthosubstituierten Arylresten.
(Aα) Es wurde nun überraschend ein Verfahren zur Herstellung von Verbindungen der Formel (I) gefunden, dadurch gekennzeichnet, dass man in einem ersten Schritt Verbindungen der Formel (X) 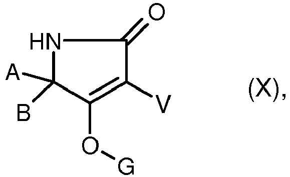 in welcher
   A und B die oben angegebenen Bedeutungen haben,
   G für die oben angegebenen Gruppen a), b), c) und E steht,
   V für Wasserstoff steht oder
(Aβ) V für COOR⁸ steht,
   wobei R⁸ für Alkyl (bevorzugt für C₁-C₈-Alkyl) steht
   und A, B und G die oben angegebenen Bedeutungen haben,
   mit einer Verbindung der Formel (XI) 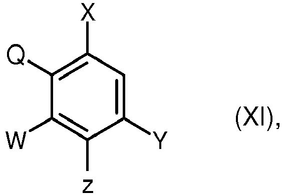 in welcher
   W, X, Y und Z die oben angegebenen Bedeutungen haben mit der Ausnahme, dass als Halogen jetzt nur noch Fluor und Chlor stehen können, X auch zusätzlich für Wasserstoff stehen darf und
   Q für Triflat, Brom oder Iod steht, bevorzugt für Brom oder Jod,
   in Gegenwart einer Base, eines Palladiumkatalysators und eines Phosphinliganden der Formel (XII') 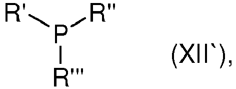 in welcher die Reste
   R', R" und R'" unabhängig voneinander für C₁ - C₁₂-Alkyl, C₅ - C₁₀- Cycloalkyl, C₆ - C₁₀-Aryl, die gegebenenfalls einfach oder mehrfach substituiert durch C₁ - C₆-Alkyl, C₁ -C₆-Alkoxy, C₁ - C₆-Alkylamino, C₁ - C₆-Dialkylamino sein können oder gegebenenfalls einfach oder mehrfach durch C₁ - C₆-Alkyl, C₁ - C₆-Alkoxy, C₁ - C₆-Alkylamino oder C₁ - C₆-Dialkylamino substituiertes Phenyl stehen,
   in einem Verdünnungsmittel umsetzt.

Als Basen kommen für das erfindunsgemäße Verfahren allgemein bekannte organische und anorganische Basen zum Einsatz. Als organische Basen seien beispielhaft Trimethylamin, Triethylamin, Tributylamin, Diisopropylamin, Diisopropylethylamin, N,N-Dimethylanilin, DABCO, DBU, Pyridin, Picoline, Luitidine, 5-Ethyl-2-methyl-pyridin genannt. Als anorganische Basen seien beispielhaft Alkali- und Erdalkalihydroxide wie LiOH, NaOH, KOH, Mg(OH)₂ und Ca(OH)₂, Alkalialkoholate wie NaOMe, NaOEt, NaOtert.butyl, KOtert.butyl, Alkali- und Erdalkalicarbonate wie Na₂CO₃, K₂CO₃, Cs₂CO₃ und CaCO₃, Alkali- und Erdalkalihydrogencarbonate wie NaHCO₃, KHCO₃, Alkali- und Erdalkaliphosphate wie Na₃PO₄, K₃PO₄ und Mg₃(PO₄)₂, Alkali- und Erdalkalihydrogenphosphate wie Na₂HPO₄, K₂HPO₄ und BaHPO₄, Alkali- und Erdalkalidihydrogenphosphate wie NaH₂PO₄, KH₂PO₄ und Ca(H₂PO₄)₂, Alkali- und Erdalkalihydride wie NaH, KH und CaH₂ und Alkali- und Erdalkaliamide wie NaNH2, KNH₂ und LiNPr₂ genannt.

Bevorzugt sind die Alkali- und Erdalkalicarbonate und -phosphate.

Im erfindunsgemäße Verfahren kann die eingesetzte Basenmenge in weiten Bereichen variiert werden. Für gewöhnlich wird man jedoch mindestens ein Moläquivalent Base bezogen auf die Verbindung der allgemeinen Formel (X) einsetzen. Es ist auch möglich, die Base in Überschüssen von 1,1 bis 15, bevorzugt 1,1 bis 6 Moläquivalenten Base bezogen auf die Verbindung der allgemeinen Formel (X) einzusetzen.

Als Palladiumkatalysatoren für das erfindunsgemäße Verfahren kommen grundsätzlich alle Palladiumverbindungen in Frage, aus denen unter den Reaktionsbedingungen *in situ* ein aktiver Katalysator enstehen kann. Beispielhaft seien hier genannt: Pdalladiumchlorid, Palladiumbromid, Palladiumiodid, Palladiumacetat, Palladiumtrifluoracetat, Palladiumnitrat, Palladiumsulfat, Palladiumacetylacetonat, Allylpalladiumchlorid-Dimer, Bis(dibenzylidenaceton)-palladium, Bis(triphenylphosphin)-palladium(II)chlorid, Bis(triphenylphosphin)palladium(II)bromid, Tetrakis(triphenylphosphin)palladium(0), Bis(acetonitril)palladiumdichlorid, Bis(benzonitril)-palladiumdichlorid, 1,1'-Bis(diphenylphosphino)ferrocen-palladiumdichlorid, Di-µ-chlorobis(tri-tert.butylphosphino)-dipalladium(I), Di-µ-bromobis(tri-tert.butylphosphino)-dipalladium(I), metallisches Palladium wie Palladiumschwarz oder Palladiumpulver, oder Palladium auf verschiedenen Trägern wie beispielsweise Palladium auf Aktivohle, Palladium auf Bariumsulfat, Palladium auf Calciumcarbonat oder Palladium auf Aluminiumoxid.

Die im erfindungsgemäßen Verfahren einzusetzende Menge an Palladiumkatalysator kann innerhalb weiter Grenzen variiert werden. Für gewöhnlich wird man die geringstmögliche Menge, mit der noch eine gute Ausbeute erzielt wird, einsetzen. Typischerweise liegt die Menge an Palladiumkatalysator zwischen 0,001 und 10 Molprozent, bezogen auf die Verbindung der allgemeinen Formel (X). Bevorzugt werden Mengen von 0,01 bis 5 Molprozent eingesetzt.

Als Verdünnungsmittel für das erfindunsgemäße Verfahren können im Prinzip alle unter den Reaktionsbedingungen inerten organischen Lösungsmittel verwendet werden. Beispielhaft seien genannt: Ether wie Diethylether, Methyl-tert.butylether, Methyl-cyclopentylether, Tetrahydrofuran, 2-Methyltetrahydrofuran, 1,4-Dioxan; Kohlenwasserstoffe wie Toluol, Xylole, Mesitylen, Chlorbenzol, 1,2-Dichlorbenzol; Amide wie N,N-Dimethylformamid, N,N-Dimethylacetamid, N-Methyl-pyrrolidon; Dimethylsulfoxid oder Sulfolan.

Im erfindungsgemäßen Verfahren können unterschiedlichste Phosphinliganden der allgemeinen Formel (XII') eingesetzt werden. Beipielhaft seien genannt: Triphenylphosphin, Tri-ortho-tolyl-phosphin, Tri-meta-tolyl-phosphin, Tri-para-tolyl-phosphin,

Benzyl-di-1-adamantylphosphin (cataCXium ABn), Dinatrium-bis(4,6-dimethyl-3-sulfonatophenyl)-(2,4dimethylphenyl)-phosphin, Trinatrium-tris(4,6-dimethyl-3-sulfonato-phenyl)phosphin, Butyl-di-1-adamantylphosphin (cataCXium A), Tributylphosphin, Tricyclohexylphosphin, Tri-tert.butylphosphin, 2-Di-tert.butylphosphino-1,1'-binaphthyl, 2-Di-tert.butylphosphino-1,1'-biphenyl, 2-Di-cyclohexylphosphino-biphenyl, 2-Di-tert.butylphosphino-2'-(N,N-dimethylamino)biphenyl, 2-Di-cyclohexylphosphino-2'-(N,N-dimethylamino)biphenyl, 2-Di-tert.butylphosphino-2'-methylbiphenyl, 2-Di-cyclohexylphosphino-2'-methylbiphenyl, 2-Di-cyclohexylphosphino-2'-isopropylbiphenyl, 2-Di-tert.butylphosphino-2',4',6'-tri-isopropyl-1,1'-biphenyl, 2-Di-cyclohexylphosphino-2',4',6'-tri-isopropyl-1,1'-biphenyl, 2-Diphenylphosphino-2'-(N,N-dimethylamino)biphenyl, 2-Dicyclohexylphosphino-2',6'-di-iso-propoxy-1,1'-biphenyl (RuPhos), N-(2-Methoxyphenyl)-2-(di-tert.butylphosphino)pyrrol, N-Phenyl- 2-(di-tert.butyl-phosphino)-pyrrol, 9,9-Dimethyl-4,5-bis(diphenylphosphino)xanthen (XANTPHOS), 9,9-Dimethyl-4,5-bis(di-tert.butylphosphino)xanthen, Bis(2-diphenylphosphinophenyl)ether (DPEphos), 2,2'-Bis(diphenylphosphino)-1,1'-biphenyl (BIPHEP), 2,2'-Bis(diphenylphosphino)-1,1'-binaphthyl (BINAP), 1,1'-Bis(diphenylphosphino)-ferrocen (DPPF).

Die im erfindungsgemäßen Verfahren einzusetzende Menge an Phosphinliganden der allgemeinen Formel (XII') liegt zwischen 0,25 und 5 Mol pro Mol Palladiumkatalysator. Bevorzugt setzt man zwischen 0,5 und 2,5 Mol pro Mol ein.

Die Reaktionstemperatur für das erfindungsgemäße Verfahren kann in weiten Grenzen variiert werden. Üblicherweise arbeitet man bei einer Temperatur zwischen 20 und 200°C, bevorzugt zwischen 50 und 180°C.

Das erfindungsgemäße Verfahren wird üblicherweise bei Normaldruck unter Ausschluß von Luftsauerstoff und Feuchtigkeit ausgeführt. Das Verfahren kann aber grunsätzlich auch unter vermindertem oder erhöhtem Druck durchgeführt werden.

Bei der Durchführung des erfindungsgemäßen Verfahrens ist es möglich, die Verbindungen der Formel (XI) in einem größeren Überschuss (bis zu 10 Mol, bevorzugt bis zu 2 Mol) einzusetzen.

Einige Verbindungen der Formel (X) mit G = Wasserstoff und substituertem Benzoyl sind teilweise bekannt aus WO 94/01401 sowie der dort angegebenen Literatur und teilweise neu.

Die Verbindungen der Formel (X) 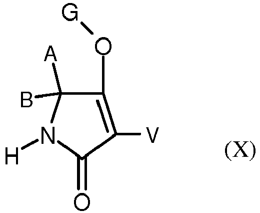 mit
- A, B: und das Kohlenstoffatom an das sie gebunden sind, stehen bevorzugt für gesättigtes C₅-C₆-Cycloalkyl, worin ein Ringglied durch Sauerstoff oder Schwefel ersetzt ist und welches gegebenenfalls einfach oder zweifach durch C₁-C₆-Alkyl oder C₁-C₆-Alkoxy substituiert ist oder
- A, B: und das Kohlenstoffatom, an das sie gebunden sind, stehen bevorzugt für C₃-C₆-Cycloalkyl, worin gegebenenfalls ein Ringglied durch Stickstoff ersetzt ist und welches einfach oder zweifach, unabhängig voneinander durch C₁-C₈-Alkyl, C₁-C₈-Alkoxy, Halogen, C₃-C₈-Alkenyloxy, C₁-C₆-Alkoxy-C₁-C₆-alkyl, C₃-C₆-Cycloalkyl-C₁-C₂-alkoxy, C₃-C₁₀-Cycloalkyl, C₁-C₈-Halogenalkyl, C₂-C₆-Halogenalkoxy oder C₁-C₆-Alkoxy-C₁-C₄-alkoxy substituiert ist, wobei die zuvor genannten Reste (ausgenommen Halogen und C₁-C₈-Halogenalkyl,) auch als N-Substituenten in Frage kommen und C₁-C₈-Alkyl nur bei einer Zweifachsubstitution erlaubt ist, oder
- A, B: und das Kohlenstoffatom, an das sie gebunden sind, stehen bevorzugt für C₅-C₆-Cycloalkyl, welches durch eine gegebenenfalls ein oder zwei nicht direkt benachbarte Sauerstoff- und/oder Schwefelatome enthaltende gegebenenfalls durch C₁-C₄-Alkyl substituierte Alkylendiyl- oder durch eine Alkylendioxyl- oder durch eine Alkylendithioyl-Gruppe substituiert ist, die mit dem Kohlenstoffatom, an das sie gebunden ist, einen weiteren fünf- bis achtgliedrigen Ring bildet,
- G: steht bevorzugt für Wasserstoff (a) oder für eine der Gruppen 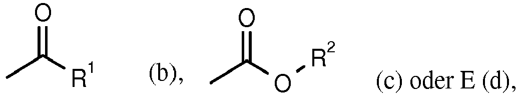 in welchen
- E: für ein Metallion oder ein Ammoniumion steht,
- R¹: steht bevorzugt für jeweils gegebenenfalls einfach bis dreifach durch Fluor oder Chlor substituiertes C₁-C₈-Alkyl, C₂-C₈-Alkenyl, C₁-C₄-Alkoxy-C₁-C₂-alkyl, C₁-C₄-Alkylthio-C₁-C₂-alkyl oder gegebenenfalls einfach bis zweifach durch Fluor, Chlor, C₁-C₂-Alkyl oder C₁-C₂-Alkoxy substituiertes C₃-C₆-Cycloalkyl, in welchem gegebenenfalls ein oder zwei nicht direkt benachbarte Ringglieder durch Sauerstoff ersetzt sind,
für gegebenenfalls einfach bis zweifach durch Fluor, Chlor, Brom, Cyano, Nitro, C₁-C₄-Alkyl, C₁-C₄-Alkoxy, C₁-C₂-Halogenalkyl oder C₁-C₂-Halogenalkoxy substituiertes Phenyl,
- R²: steht bevorzugt für jeweils gegebenenfalls einfach bis dreifach durch Fluor substituiertes C₁-C₈-Alkyl, C₂-C₈-Alkenyl oder C₁-C₄-Alkoxy-C₂-C₄-alkyl,
für gegebenenfalls einfach durch C₁-C₂-Alkyl oder C₁-C₂-Alkoxy substituiertes C₃-C₆-Cycloalkyl oder
für jeweils gegebenenfalls einfach bis zweifach durch Fluor, Chlor, Brom, Cyano, Nitro, C₁-C₄-Alkyl, C₁-C₃-Alkoxy, Trifluormethyl oder Trifluormethoxy substituiertes Phenyl oder Benzyl,
- V: steht bevorzugt für Wasserstoff (X-1) oder COOR⁸ (X-2),
in welcher R⁸ bevorzugt für C₁-C₈-Alkyl steht,
sind neu.

Unter Einbeziehung der verschiedenen Bedeutungen (a), (b), (c) und (d) der Gruppe G ergeben sich folgende hauptsächliche Strukturen (X-1-a) bis (X-1-d), wenn V für Wasserstoff steht, 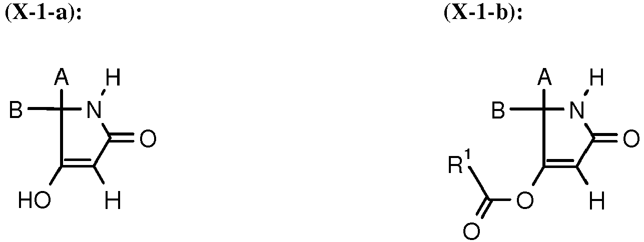 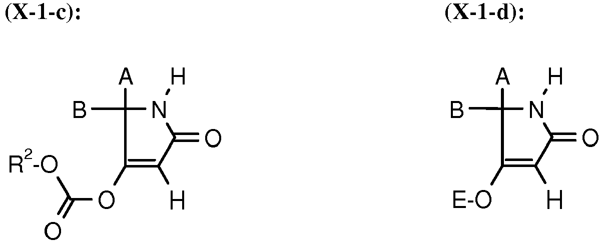 worin
A, B, E, R¹ und R² die oben angegebenen Bedeutungen besitzen.

Unter Einbeziehung der verschiedenen Bedeutungen (a), (b), (c) und (d) der Gruppe G ergeben sich folgende hauptsächliche Strukturen (X-2-a) bis (X-2-d), wenn V für COOR⁸ steht, 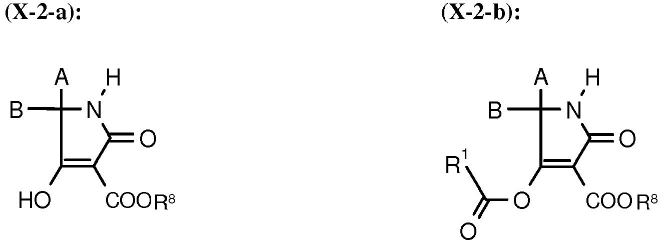 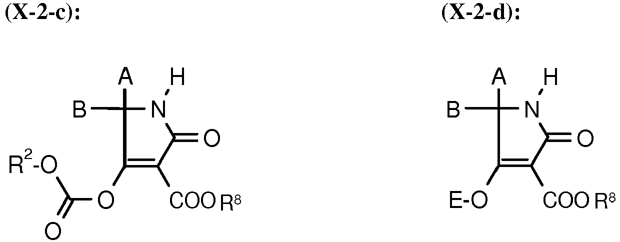 worin
A, B, E, R¹, R² und R⁸ die oben angegebenen Bedeutungen besitzen.

Die erfindungsgemäßen Verbindungen sind durch die Formel (X) allgemein definiert. Bevorzugte Substituenten bzw. Bereiche der in der oben und nachstehend erwähnten Formeln aufgeführten Reste werden im Folgenden erläutert:
- A, B: und das Kohlenstoffatom an das sie gebunden sind, stehen besonders bevorzugt für gesättigtes C₅-C₆-Cycloalkyl, worin ein Ringglied durch Sauerstoff oder Schwefel ersetzt ist und welches gegebenenfalls einfach bis zweifach durch C₁-C₄-Alkyl oder C₁-C₄-Alkoxy substituiert ist oder
- A, B: und das Kohlenstoffatom an das sie gebunden sind, stehen besonders bevorzugt für gesättigtes C₃-C₇-Cycloalkyl, worin gegebenenfalls ein Ringglied durch Stickstoff, ersetzt ist und welches einfach bis zweifach, unabhängig voneinander durch C₁-C₆-Alkyl, Chlor, Fluor, C₁-C₄-Alkoxy-C₁-C₂-alkyl, Trifluormethyl, C₁-C₆-Alkoxy, C₃-C₆-Alkenyloxy, Trifluorethoxy, C₁-C₃-Alkoxy-C₁-C₃-alkoxy oder C₃-C₆-Cycloalkylmethoxy substituiert ist, wobei die zuvor genannten Reste (ausgenommen Fluor, Chlor, Trifluormethyl) auch als N-Substituenten in Frage kommen und C₁-C₆-Alkyl nur bei einer Zweifachsubstitution erlaubt ist, oder
- A, B: und das Kohlenstoffatom, an das sie gebunden sind, stehen besonders bevorzugt für gesättigtes C₅-C₆-Cycloalkyl, welches durch eine gegebenenfalls ein oder zwei nicht direkt benachbarte Sauerstoff- oder Schwefelatome enthaltende gegebenenfalls einfach oder zweifach durch Methyl oder Ethyl substituierte Alkylendiyl- oder durch eine Alkylendioxyl- oder durch eine Alkylendithiol-Gruppe substituiert ist, die mit dem Kohlenstoffatom, an das sie gebunden ist, einen weiteren fünf- oder sechsgliedrigen Ring bildet,
- G: steht besonders bevorzugt für Wasserstoff (a) oder für eine der Gruppen 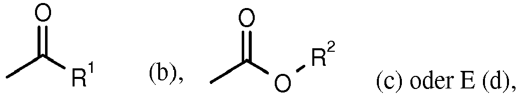 in welchen
- E: besonders bevorzugt für ein Metallion oder ein Ammoniumion steht,
- R¹: steht besonders bevorzugt für jeweils gegebenenfalls einfach durch Chlor substituiertes C₁-C₆-Alkyl, C₂-C₆-Alkenyl, C₁-C₂-Alkoxy-C₁-alkyl, C₁-C₂-Alkylthio-C₁-alkyl oder jeweils gegebenenfalls einfach durch Fluor, Chlor, Methyl oder Methoxy substituiertes Cyclopropyl oder Cyclohexyl,
für gegebenenfalls einfach durch Fluor, Chlor, Brom, Cyano, Nitro, Methyl, Methoxy, Trifluormethyl oder Trifluormethoxy substituiertes Phenyl,
- R²: steht besonders bevorzugt für jeweils gegebenenfalls einfach durch Fluor substituiertes C₁-C₈-Alkyl, C₂-C₆-Alkenyl oder C₁-C₄-Alkoxy-C₂-C₃-alkyl, Phenyl oder Benzyl,
- V: steht besonders bevorzugt für Wasserstoff oder COOR⁸, in welcher R⁸ besonders bevorzugt für C₁-C₆-Alkyl steht.
- A, B: und das Kohlenstoffatom an das sie gebunden sind, stehen ganz besonders bevorzugt für gesättigtes C₅-C₆-Cycloalkyl, in welchem ein Ringglied durch Sauerstoff ersetzt ist und welches gegebenenfalls einfach durch Methyl, Ethyl, Methoxy oder Ethoxy, substituiert ist, oder
- A, B: und das Kohlenstoffatom an das sie gebunden sind, stehen ganz besonders bevorzugt für gesättigtes C₅-C₆-Cycloalkyl, in welchem gegebenenfalls ein Ringglied durch Stickstoff, ersetzt ist und welches einfach oder zweifach, unabhängig voneinander durch Methyl, Ethyl, Trifluormethyl, Fluor, Methoxymethyl, Ethoxymethyl, Methoxyethyl, Ethoxyethyl, Methoxy, Ethoxy, Propoxy, Butoxy, Methoxyethoxy, Ethoxyethoxy, Allyloxy, Trifluorethoxy oder Cyclopropylmethoxy substituiert ist, wobei die zuvor genannten Reste (ausgenommen Fluor und Trifluormethyl) auch als N-Substituenten in Frage kommen und Methyl oder Ethyl nur bei einer Zweifachsubstitution erlaubt ist und oder
- A, B: und das Kohlenstoffatom, an das sie gebunden sind, stehen ganz besonders bevorzugt für C₆-Cycloalkyl, welches gegebenenfalls durch eine gegebenenfalls durch ein Sauerstoffatom unterbrochene Alkylidendiyl-Gruppe oder durch eine mit zwei nicht direkt benachbarten Sauerstoffatomen enthaltende Alkylendioxy-Gruppe substituiert ist, wobei ein 5- oder 6-Ringketal gebildet wird, die jeweils gegebenenfalls einfach oder zweifach durch Methyl substituiert sein können,
- G: steht ganz besonders bevorzugt für Wasserstoff (a) oder für eine der Gruppen 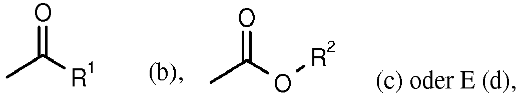 in welchen
- E: ganz besonders bevorzugt für ein Li⁺, Na⁺, K⁺ oder Cs⁺ steht,
- R¹: steht ganz besonders bevorzugt für Methyl, Ethyl, Propyl, iso-Propyl, Butyl, iso-Butyl, sec.-Butyl, tert.-Butyl, Cyclopropyl oder Cyclohexyl,
- R²: steht ganz besonders bevorzugt für Methyl, Ethyl, Propyl, iso-Propyl, Butyl, iso-Butyl, sec.-Butyl, tert.-Butyl, Phenyl oder Benzyl,
- V: steht ganz besonders bevorzugt für Wasserstoff oder COOR⁸,
in welcher R⁸ ganz besonders bevorzugt für Methyl, Ethyl, Propyl, iso-Propyl, Butyl, iso-Butyl, sec.-Butyl, tert.-Butyl steht.
- A, B: und das Kohlenstoffatom an das sie gebunden sind, stehen hervorgehoben für gesättigtes C₆-Cycloalkyl, worin ein Ringglied durch Sauerstoff ersetzt ist oder
- A, B: und das Kohlenstoffatom an das sie gebunden sind, stehen hervorgehoben für gesättigtes C₆-Cycloalkyl, welches zweifach durch Fluor oder einfach durch Methoxy substituiert ist, oder
- A, B: und das Kohlenstoffatom, an das sie gebunden sind, stehen hervorgehoben für C₆-Cycloalkyl, welches durch eine mit zwei nicht direkt benachbarte Sauerstoffatome enthaltende Alkylendiyl-Gruppe substituiert ist, die mit dem Kohlenstoffatom, an das sie gebunden ist, einen weiteren fünfgliedrigen Ring bildet,
- G: steht hervorgehoben für Wasserstoff (a) oder 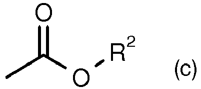 wobei R² hervorgehoben für Ethyl steht,
- V: steht hervorgehoben für Wasserstoff (X-1) oder COOR⁸ (X-2),
wobei R⁸ hervorgehoben für Methyl steht.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (X), in welchen eine Kombination der vorstehend als bevorzugt (vorzugsweise) aufgeführten Bedeutungen vorliegt.

Erfindungsgemäß besonders bevorzugt werden die Verbindungen der Formel (X), in welchen eine Kombination der vorstehend als besonders bevorzugt aufgeführten Bedeutungen vorliegt.

Erfindungsgemäß ganz besonders bevorzugt werden die Verbindungen der Formel (X), in welchen eine Kombination der vorstehend als ganz besonders bevorzugt aufgeführten Bedeutungen vorliegt.

Hervorgehoben sind Verbindungen der Formel (X) in welchen G für Wasserstoff steht.

Gesättigte oder ungesättigte Kohlenwasserstoffreste wie Alkyl, Alkandiyl oder Alkenyl können, auch in Verbindung mit Heteroatomen, wie z.B. in Alkoxy, soweit möglich, jeweils geradkettig oder verzweigt sein.

Gegebenenfalls substituierte Reste können, sofern nicht anderes angegeben ist, einfach oder mehrfach substituiert sein, wobei bei Mehrfachsubstitutionen die Substituenten gleich oder verschieden sein können.

Im Einzelnen seien außer bei den Beispielen genannten Verbindungen die folgenden Verbindungen der Formle (X) mit G = H genannt:

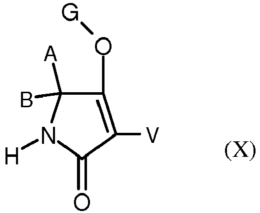

**Tabelle 1**

| **A** | **B** | **V** |
|---|---|---|
| -(CH₂)₂-O-(CH₂)₂- | | H |
| -CH₂-O-(CH₂)₃- | | H |
| -(CH₂)₂-S-(CH₂)₂- | | H |
| -CH₂-CHCH₃-(CH₂)₃- | | H |
| -CH₂-CHOCH₃-(CH₂)₂- | | H |
| -CH₂-CHOC₂H₅-(CH₂)₂- | | H |
| -CH₂-CHOC₃H₇-(CH₂)₂- | | H |
| -CH₂-CHOC₄H₉-(CH₂)₂- | | H |
| -CH₂-CHO(CH₂)₂OCH₃-(CH₂)₂- | | H |
| 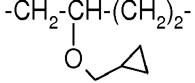 | | H |
| -CH₂-CHOCH₃-(CH₂)₃- | | H |
| -CH₂-CHOC₂H₅-(CH₂)₃- | | H |
| -CH₂-CHOC₃H₇-(CH₂)₃- | | H |
| -CH₂-CHOC₄H₉-(CH₂)₃- | | H |
| -CH₂-CHO(CH₂)₂OCH₃-(CH₂)₃- | | H |
| 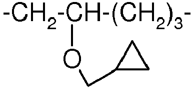 | | H |
| -(CH₂)₂-CHOCH₃-(CH₂)₂- | | H |
| -(CH₂)₂-CHOC₂H₅-(CH₂)₂- | | H |
| -(CH₂)₂-CHOC₃H₇-(CH₂)₂- | | H |
| -(CH₂)₂-CHO-CH₂CF₃-(CH₂)₂- | | H |
| -(CH₂)₂-C(CH₃)₂-(CH₂)₂- | | H |
| -CH₂-(CHCH₃)₂-(CH₂)₂- | | H |
| -(CH₂)₂-CF₂-(CH₂)₂- | | H |
| 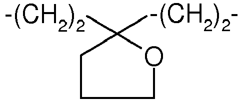 | | H |
| 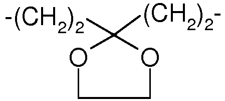 | | H |
| 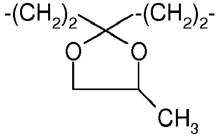 | | H |
| 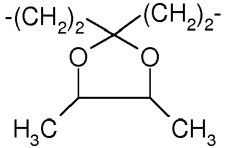 | | H |
| 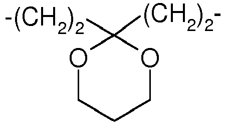 | | H |
| 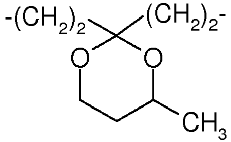 | | H |
| 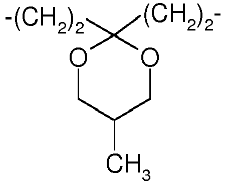 | | H |
| 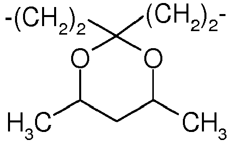 | | H |
| 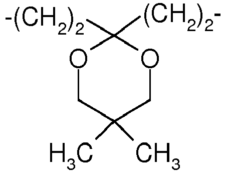 | | H |
| 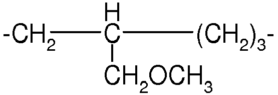 | | H |
| 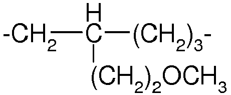 | | H |
| 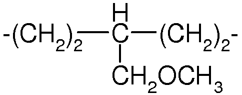 | | H |
| 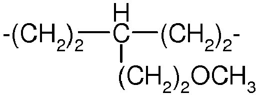 | | H |
| 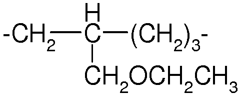 | | H |
| 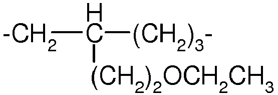 | | H |
| 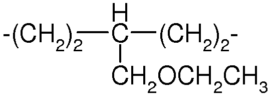 | | H |
| 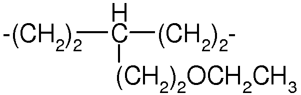 | | H |

**Tabelle 2** A und B wie in Tabelle 1 angegeben und V = COOCH₃
**Tabelle 3** A und B wie in Tabelle 1 angegeben und V = COOC₂H₅

### (B) Man erhält Verbindungen der Formel (X-2-a)

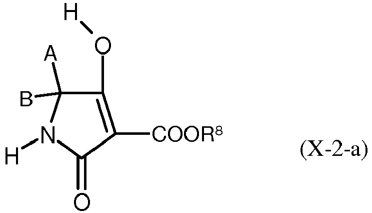

in welcher A, B und R8 die oben angegebenen Bedeutungen haben,
wenn man
Verbindungen der Formel (XII) 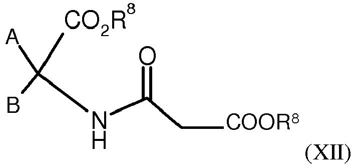 in welcher
A, B und R⁸ die oben angegebenen Bedeutungen haben,
in Gegenwart eines Verdünnungsmittels und in Gegenwart einer Base intramolekular kondensiert.

### (C) Außerdem erhält man Verbindungen der Formel (X-1-a)

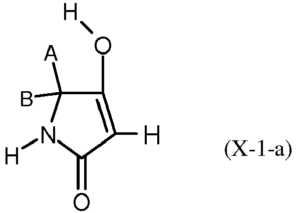

in welcher
A und B die oben angegebenen Bedeutungen haben,
wenn man
Verbindungen der Formel (X-2-a) 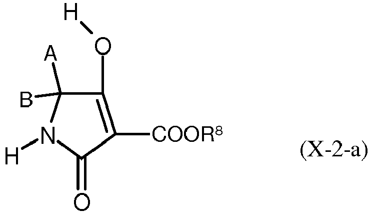 in welcher A, B und R⁸ die oben angegebenen Bedeutungen haben, hydrolysiert und anschließend decarboxyliert.

Weiterhin wurde gefunden
- (D): dass man die Verbindungen der oben gezeigten Formeln (X-1-b) oder (X-2-b), in welchen A, B und V die oben angegebenen Bedeutungen haben, erhält, wenn man Verbindungen der oben gezeigten Formeln (X-1-a) und (X-2-a), in welchen A, B und V die oben angegebenen Bedeutungen haben, jeweils
- α): mit Verbindungen der Formel (III) 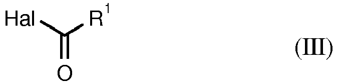 in welcher
- R¹: die oben angegebene Bedeutung hat und
- Hal: für Halogen (insbesondere Chlor oder Brom) steht
oder
- β): mit Carbonsäureanhydriden der Formel (IV)

R¹-CO-O-CO-R¹ (IV)

in welcher
- R¹: die oben angegebene Bedeutung hat,
gegebenenfalls in Gegenwart eines Verdünnungsmittels und gegebenenfalls in Gegenwart eines Säurebindemittels umsetzt;
- (E): dass man die Verbindungen der oben gezeigten Formeln (X-1-c) oder (X-2-c), in welchen R², A, B und V die oben angegebenen Bedeutungen haben, erhält, wenn man Verbindungen der oben gezeigten Formeln (X-1-a) und (X-2-a), in welchen A, B und V die oben angegebenen Bedeutungen haben,
mit Chlorameisensäureestern der Formel (V)

R²-O-CO-Cl (V)

in welcher
- R²: die oben angegebene Bedeutung hat,
gegebenenfalls in Gegenwart eines Verdünnungsmittels und gegebenenfalls in Gegenwart eines Säurebindemittels umsetzt;
- (F): dass man Verbindungen der oben gezeigten Formeln (X-1-d) und (X-2-d), in welchen E, A, B und V die oben angegebenen Bedeutungen haben, erhält, wenn man Verbindungen der Formeln (X-1-a) und (X-1-b), in welchen A, B und V die oben angegebenen Bedeutungen haben, jeweils
mit Metallverbindungen oder Aminen der Formeln (XIII) oder (XIV)

Me(OR¹⁰)ₜ (XIII)

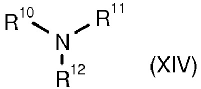

in welchen
- Me: für ein ein- oder zweiwertiges Metall (bevorzugt ein Alkali- oder Erdalkalimetall wie Lithium, Natrium, Kalium, Cäsium, Magnesium oder Calcium),
- t: für die Zahl 1 oder 2 und
- R¹⁰, R¹¹, R¹²: unabhängig voneinander für Wasserstoff oder Alkyl (bevorzugt C₁-C₈-Alkyl) stehen, wobei im Falle von Me R¹⁰ auch für die Gruppe COO oder HCOO stehen kann
gegebenenfalls in Gegenwart eines Verdünnungsmittels umsetzt.

Verwendet man beispielsweise gemäß Verfahren (Aα) 8-Methoxy-1-azaspiro[4,5]decan-2,4-dion und 2,5-Dimethyl-brombenzol als Ausgangsstoffe, so kann der Reaktionsverlauf durch folgendes Schema wiedergegeben werden:

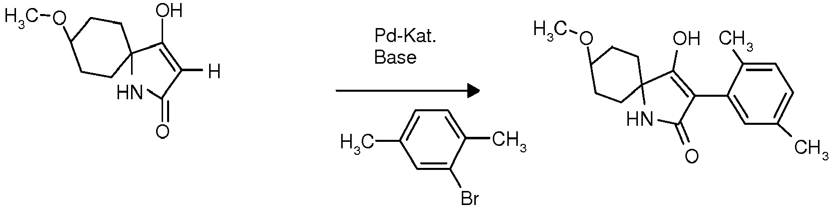

Verwendet man beispielsweise gemäß Verfahren (Aß) 3-Methoxycarbonyl-8-methoxy-1-azaspiro-[4,5]-decan-2,4-dion und 2,5-Dimethyl-brombenzol als Ausgangsverbindungen, so kann der Verlauf des erfindungsgemäßen Verfahrens durch folgendes Reaktionsschema wiedergegeben werden:

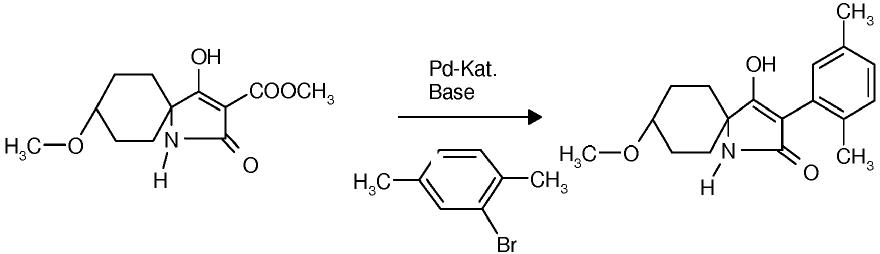

Verwendet man beispielsweise gemäß Verfahren (B) N-Ethoxycarbonylacetyl-1-amino-4-methoxy-cyclohexancarbonsäureethylester als Ausgangsstoff, so kann der Verlauf des erfindungsgemäßen Verfahrens durch folgendes Reaktionsschema wiedergegeben werden:

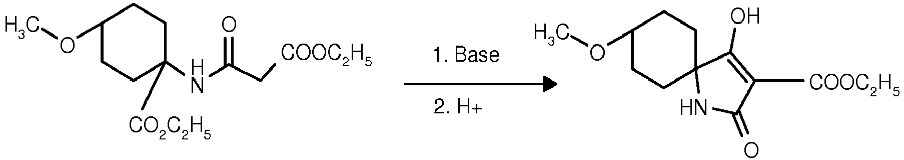

Verwendet man beispielsweise gemäß Verfahren (C) 3-Methoxycarbonyl-8-methoxy-1-azaspiro[4,5]decan-2,4-dion und einen Überschuss an wässriger Base als Ausgangsprodukte, so kann der Reaktionsverlauf durch folgendes Reaktionsschema wiedergegeben werden:

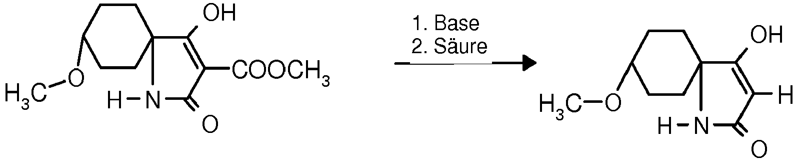

Verwendet man beispielsweise gemäß Verfahren (Dα) 8-Methoxy-1-azaspiro[4,5]decan-2,4-dion und Acetylchlorid als Ausgangsstoffe, so kann der Verlauf des erfindungsgemäßen Verfahrens durch folgendes Reaktionsschema wiedergegeben werden:

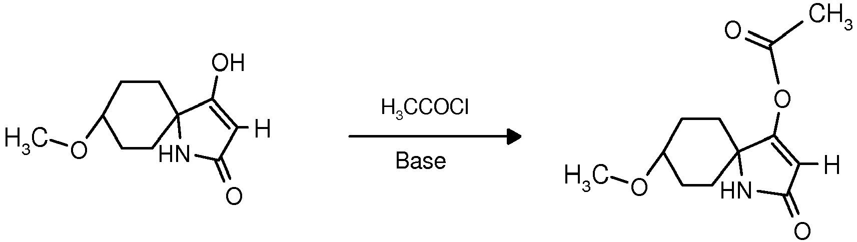

Verwendet man beispielsweise gemäß Verfahren (D β) 8-Methoxy-1-azaspiro-[4,5]-decan-2,4-dion und Acetanhydrid als Ausgangsverbindungen, so kann der Verlauf des erfindungsgemäßen Verfahrens durch folgendes Reaktionsschema wiedergegeben werden:

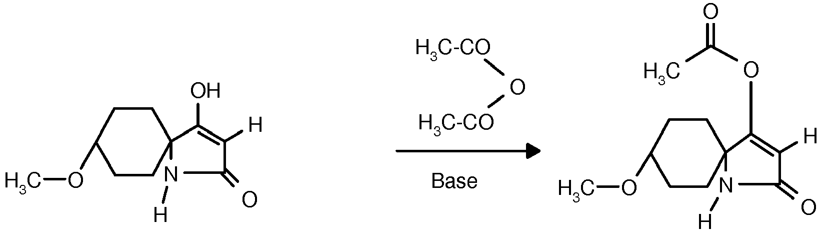

Verwendet man beispielsweise gemäß Verfahren (E) 8-Methoxy-1-azaspiro[4,5]decan-2,4-dion und Chlorameisensäureethylester als Ausgangsverbindungen, so kann der Verlauf des erfindungsgemäßen Verfahrens durch folgendes Reaktionsschema wiedergegeben werden:

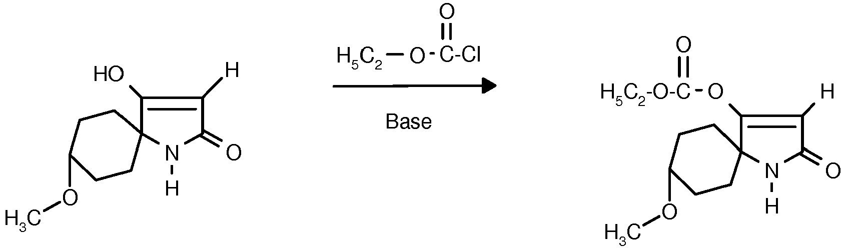

Verwendet man beispielsweise gemäß Verfahren (F) 8-Methoxy-1-azaspiro[4,5]decan-2,4-dion und beispielsweise Natriumhydroxid (equimolar) als Ausgangsprodukte, so kann der Reaktionsverlauf folgendermaßen wiedergegeben werden:

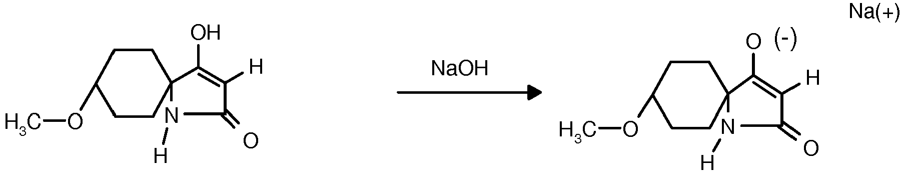

Die beim erfindungsgemäßen Verfahren (B) als Ausgangsstoffe benötigten Verbindungen der Formel (XII) 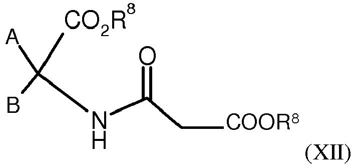 in welcher
A, B und R⁸ die oben angegebenen Bedeutungen haben,
sind neu.

Man erhält die Acylaminosäureester der Formel (XII) beispielsweise, wenn man Aminosäurederivate der Formel (XV) 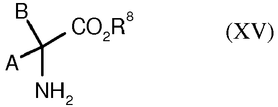 in welcher
A, B und R⁸ die oben angegebene Bedeutung haben,
mit substituierten Malonsäurehalbesterchloriden der Formel (XVI) 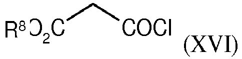 in welcher R⁸ die oben angegebenen Bedeutungen hat,
acyliert (Chem. Reviews 52, 237-416 (19953); Bhattacharya, Indian J. Chem. 6, 341-5, 1968).

Weiterhin lassen sich die bei dem obigen Verfahren (B) verwendeten Ausgangsstoffe der Formel (XII) 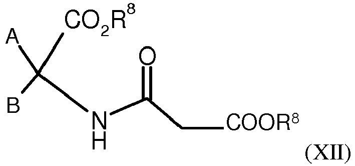 in welcher
A, B und R⁸ die oben angegebenen Bedeutungen haben,
herstellen, wenn man 1-Amino-carbonsäurenitrile der Formel (XVII) 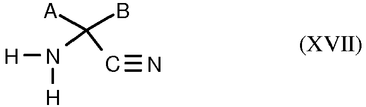 in welcher
A und B die oben angegebenen Bedeutungen haben,
mit Malonsäurehalbesterchloriden der Formel (XVI) 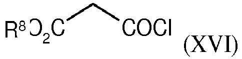 in welcher R⁸ die oben angegebene Bedeutungen hat,
zu Verbindungen der Formel (XVIII) 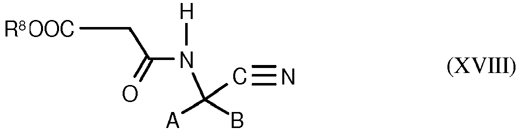 in welcher
A, B und R⁸ die oben angegebenen Bedeutungen haben,
umsetzt,
und diese anschließend einer sauren Alkoholyse unterwirft.

Die Verbindungen der Formel (XVIII) sind ebenfalls neu und lassen sich analog nach bekannten Verfahren die in der eingangs zitierten Literatur oder z.B. wie in EP-A-595 130 beschrieben sind herstellen. Die Verbindungen der Formel (XVII) sind teilweise käuflich, teilweise bekannt z.B. WO 2008/128058 und teilweise auch neu und lassen sich z.B. wie in EP-A-595 130 beschrieben herstellen.

Außerdem lassen sich die bei dem obigen Verfahren (B) verwendeten Ausgangsstoffe der Formel (XII) 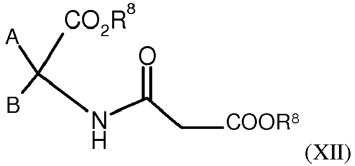 in welcher
A, B, und R⁸ die oben angegebenen Bedeutungen haben,
herstellen, wenn man 1-Aminocarbonsäurenitrile der Formel (XVII) 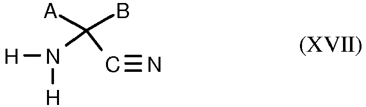 in welcher
A und B die oben angegebenen Bedeutungen haben,
mit Cyanessigsäure der Formel (XIX) 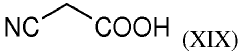 zu Verbindungen der Formel (XX) 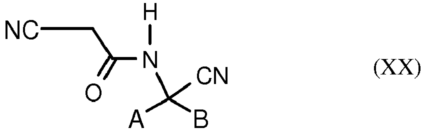 in welcher
A und B die oben angegebenen Bedeutungen haben,
umsetzt,
und diese anschließend einer sauren Alkoholyse unterwirft.

Die Verbindungen der Formel (XX) sind ebenfalls neu und lassen sich analog nach bekannten Verfahren die in der eingangs zitierten Literatur beschrieben sind herstellen.

Die zur Durchführung der erfindungsgemäßen Verfahren (D), (E) und (F) außerdem als Ausgangsstoffe benötigten Säurehalogenide der Formel (III), Carbonsäureanhydride der Formel (IV), Chlorameisensäureester der Formel (V) und Metallhydroxide, Metallalkoxide, Metallcarbonate, Metallhydrogencarbonate oder Amine der Formel (XIII) und (XIV) sind allgemein bekannte Verbindungen der organischen bzw. anorganischen Chemie.

Die Verbindungen der Formeln (XV) und (XVII) sind darüber hinaus aus den eingangs zitierten Patentanmeldungen bekannt und/oder lassen sich nach den dort angegebenen Methoden herstellen.

Die Verbindungen der Formeln (XVI) und (XIX) sind käuflich.

Das Verfahren (B) ist dadurch gekennzeichnet, dass man Verbindungen der Formel (XII), in welcher A, B und R⁸ die oben angegebenen Bedeutungen haben, in Gegenwart eines Verdünnungsmittels und in Gegenwart einer Base einer intramolekularen Kondensation unterwirft.

Als Verdünnungsmittel können bei dem erfindungsgemäßen Verfahren (B) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (B) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (=Methyltrialkyl(C₈-C₁₀)ammoniumchlorid) oder TDA 1 (=Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natriummethylat, Natriumethylat und Kalium-tert.-butylat einsetzbar.

Die Reaktionstemperatur kann bei der Durchführung des erfindungsgemäßen Verfahrens (B) innerhalb eines größeren Bereiches variiert werden. Im allgemeinen arbeitet man bei Temperaturen zwischen -75°C und 200°C, vorzugsweise zwischen -50°C und 150°C. Das erfindungsgemäße Verfahren (A) wird im allgemeinen unter Normaldruck durchgeführt.

Bei der Durchführung des erfindungsgemäßen Verfahrens (B) setzt man die Reaktionskomponente der Formel (XII) und die deprotonierende Base im allgemeinen in äquimolaren bis etwa doppeltäquimolaren Mengen ein. Es ist jedoch auch möglich, die eine oder andere Komponente in einem größeren Überschuss (bis zu 3 Mol) zu verwenden.

Das Verfahren (C) ist dadurch gekennzeichnet, dass man Verbindungen der Formel (X-2), in welcher A, B und R⁸ die oben angegebenen Bedeutungen haben, in Gegenwart eines Verdünnungsmittels und gegebenenfalls in Gegenwart einer Base oder Säure hydrolysiert und decarboxyliert.

Als Verdünnungsmittel können bei dem erfindungsgemäßen Verfahren (C) alle gegenüber den Reaktionsteilnehmern inerten organischen Solventien eingesetzt werden. Vorzugsweise verwendbar sind Ether, wie Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Iso-Propanol, Butanol, Iso-Butanol und tert.-Butanol, aber auch Wasser.

Als Base können bei der Durchführung des erfindungsgemäßen Verfahrens (C) alle üblichen laugenbildenden Basen eingesetzt werden. Vorzugsweise verwendbar sind Alkalimetall- und Erdalkalimetalloxide, -hydroxide und -carbonate, wie Lithiumhydroxid, Natriumhydroxid, Kaliumhydroxid, Magnesiumoxid, Calciumoxid, Natriumcarbonat, Kaliumcarbonat und Calciumcarbonat einsetzbar.

Als Säuren können bei der Durchführung des erfindungsgemäßen Verfahrens (C) alle üblichen anorganischen und organischen Säuren eingesetzt werden. Vorzugsweise verwendbar sind als anorganische Säuren z.B. Salzsäure, Schwefelsäure, Phosphorsäure und Salpetersäure. Vorzugsweise verwendbar sind als organische Säuren z.B. Ameisensäure, Essigsäure, Trifluoressigsäure, Oxalsäure, Zitronensäure und deren wässrige Lösungen.

Als Besonderheit können die bei dem Verfahren (C) eingesetzten Verbindungen der Formel (X-2) auch autokatalytisch als Säure eingesezt werden.

Die Reaktionstemperatur kann bei der Durchführung des erfindungsgemäßen Verfahrens (C innerhalb eines größeren Bereiches variiert werden. Im allgemeinen arbeitet man bei Temperaturen zwischen -20°C und 200°C, vorzugsweise zwischen 0°C und 150°C. Das erfindungsgemäße Verfahren (C) wird im allgemeinen unter Normaldruck durchgeführt.

Bei der Durchführung des erfindungsgemäßen Verfahrens (C) setzt man die Reaktionskomponente der Formel (X-2) und die Base oder die Säure im allgemeinen in äquimolaren bis etwa doppeltäquimolaren Mengen ein. Es ist jedoch auch möglich, die Base oder die Säure in einem größeren Überschuss aber auch katalytisch zu verwenden.

Das Verfahren (D_{α}) ist dadurch gekennzeichnet, dass man Verbindungen der Formeln (X-1) oder (X-2) jeweils mit Carbonsäurehalogeniden der Formel (III) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (D_{α}) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylformamid, Dimethylsulfoxid und Sulfolan. Wenn die Hydrolysestabilität des Säurehalogenids es zulässt, kann die Umsetzung auch in Gegenwart von Wasser durchgeführt werden.

Als Säurebindemittel kommen bei der Umsetzung nach dem erfindungsgemäßen Verfahren (D_{α}) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperatur kann bei dem erfindungsgemäßen Verfahren (D_{α}) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (D_{α}) werden die Ausgangsstoffe der Formeln (X-1) oder (X-2) und das Carbonsäurehalogenid der Formel (III) im allgemeinen jeweils in angenähert äquivalenten Mengen verwendet. Es ist jedoch auch möglich, das Carbonsäurehalogenid in einem größeren Überschuss (bis zu 5 Mol) einzusetzen. Die Aufarbeitung erfolgt nach üblichen Methoden.

Das Verfahren (D_{β}) ist dadurch gekennzeichnet, dass man Verbindungen der Formeln (X-1) oder (X-2) jeweils mit Carbonsäureanhydriden der Formel (IV) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (D_{β}) vorzugsweise diejenigen Verdünnungsmittel verwendet werden, die auch bei der Verwendung von Säurehalogeniden vorzugsweise in Betracht kommen. Im übrigen kann auch ein im Überschuss eingesetztes Carbonsäureanhydrid gleichzeitig als Verdünnungsmittel fungieren.

Als gegebenenfalls zugesetzte Säurebindemittel kommen beim Verfahren (D_{β}) vorzugsweise diejenigen Säurebindemittel in Frage, die auch bei der Verwendung von Säurehalogeniden vorzugsweise in Betracht kommen.

Die Reaktionstemperatur kann bei dem erfindungsgemäßen Verfahren (D_{β}) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (D_{β}) werden die Ausgangsstoffe der Formeln (X-1) oder (X-2) und das Carbonsäureanhydrid der Formel (IV) im allgemeinen in jeweils angenähert äquivalenten Mengen verwendet. Es ist jedoch auch möglich, das Carbonsäureanhydrid in einem größeren Überschuss (bis zu 5 Mol) einzusetzen. Die Aufarbeitung erfolgt nach üblichen Methoden.

Im allgemeinen geht man so vor, dass man Verdünnungsmittel und im Überschuss vorhandenes Carbonsäureanhydrid sowie die entstehende Carbonsäure durch Destillation oder durch Waschen mit einem organischen Lösungsmittel oder mit Wasser entfernt.

Das Verfahren (E) ist dadurch gekennzeichnet, dass man Verbindungen der Formeln (X-1) oder (X-2) jeweils mit Chlorameisensäureestern der Formel (V) gegebenenfalls in Gegenwart eines Verdünnungsmittels und gegebenenfalls in Gegenwart eines Säurebindemittels umsetzt.

Als Säurebindemittel kommen bei dem erfindungsgemäßen Verfahren (E) alle üblichen Säureakzeptoren in Betracht. Vorzugsweise verwendbar sind tertiäre Amine, wie Triethylamin, Pyridin, DABCO, DBU, DBN, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (E) alle gegenüber den Chlorameisensäureestern inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, außerdem Nitrile wie Acetonitril und auch stark polare Solventien, wie Dimethylformamid, Dimethylsulfoxid und Sulfolan.

Die Reaktionstemperatur kann bei der Durchführung des erfindungsgemäßen Verfahrens (E) innerhalb eines größeren Bereiches variiert werden. Die Reaktionstemperatur liegt im allgemeinen zwischen -20°C und +100°C, vorzugsweise zwischen 0°C und 50°C.

Das erfindungsgemäße Verfahren (E) wird im allgemeinen unter Normaldruck durchgeführt.

Bei der Durchführung des erfindungsgemäßen Verfahrens (E) werden die Ausgangsstoffe der Formeln (X-1) oder (X-2) und der entsprechende Chlorameisensäureester der Formel (V) im Allgemeinen jeweils in angenähert äquivalenten Mengen verwendet. Es ist jedoch auch möglich, die eine oder andere Komponente in einem größeren Überschuss (bis zu 2 Mol) einzusetzen. Die Aufarbeitung erfolgt nach üblichen Methoden. Im allgemeinen geht man so vor, dass man ausgefallene Salze entfernt und das verbleibende Reaktionsgemisch durch Abziehen des Verdünnungsmittels einengt.

Das Verfahren (F) ist dadurch gekennzeichnet, dass man Verbindungen der Formeln (X-1) oder (X-2) jeweils mit Metallamiden, Metallhydriden, Metallhydroxiden, Metallalkoxiden, Metallcarbonaten oder Metallhydrogencarbonaten der Formel (XIII) oder Aminen der Formel (XIV), die beim erfindungsgemäßen Verfahren (A) genannt sind, gegebenenfalls in Gegenwart eines Verdünnungsmittels, umsetzt.

Als Verdünnungsmittel können bei dem erfindungsgemäßen Verfahren (F) vorzugsweise die beim Verfahren (A) genannten Lösungsmittel aber auch Alkohole wie Methanol, Ethanol, Isopropanol, sowie Wasser eingesetzt werden. Das erfindungsgemäße Verfahren (F) wird im allgemeinen unter Normaldruck durchgeführt. Die Reaktionstemperatur liegt im allgemeinen zwischen -20°C und 100°C, vorzugsweise zwischen 0°C und 50°C

### Herstellungsbeispiele

Anmerkung: Me steht für Methyl; Et steht für Ethyl

### Beispiel 1: 5,5-Dimethyl-3-phenylpyrrolidin-2,4-dion

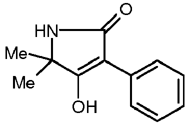

Unter Argon werden in einer ausgeheizten Apparatur 23 mg Pd(OAc)₂, 69 mg Di-tert-butyl(2'-methylbiphenyl-2-yl)phosphin und 2,44 g K₃PO₄ in 15 ml luftfreiem Dioxan vorgelegt. Man gibt 763 mg 5,5-Dimethylpyrrolidin-2,4-dion und 785 mg Brombenzol hinzu und rührt 16 Stunden unter Rückfluß. Danach lässt man auf Raumtemperatur abkühlen, verdünnt mit 20 ml Methanol, filtriert und wäscht den Filterrückstand mit 10 ml MeOH nach. Die vereinigten Filtrate werden am Rotationsverdampfer eingeengt. Der Rückstand wird in 20 ml Wasser aufgenommen und mit verdünnter Salzsäure schwach sauer gestellt. Der ausgefallene Feststoff wird abgesaugt und mit 10 ml Wasser gewaschen. Dann wird er mit Aceton vom Filter gewaschen und das Filtrat eingeengt. Man erhält 1,01 g Feststoff mit einer Reinheit von 93,8% nach GC/MS.
GC/MS: m/e = 203 (M⁺, 20%), 118 (M - NHCMe₂CO, 100%).
¹H-NMR (400 MHz, d-DMSO): δ = 1,35 (s, 6H), 7,13-7,17 (m, 1H), 7,28-7,32 (m, 2H), 7,65 (s, 1H), 7,91-7,93 (m, 2H), 11,08 (s, 1H) ppm.

### Beispiel 2: 5,5-Dimethyl-3-(2-methylphenyl)pyrrolidin-2,4-dion

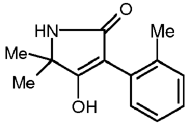

Man verfährt wie in Beispiel 1, mit dem Unterschied, dass 855 mg 2-Bromtoluol anstelle von Brombenzol eingesetzt werden. Man erhält 0,84 g Feststoff mit einer Reinheit laut GC/MS von 86,3%.
GC/MS: m/e = 217 (M⁺, 30%), 132 (M - NHCMe₂CO, 100%).

### Beispiel 3: 5,5-Dimethyl-3-(3-methylphenyl)pyrrolidin-2,4-dion

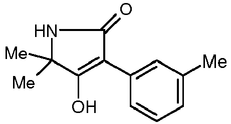

Man verfährt wie in Beispiel 1, mit dem Unterschied, dass 855 mg 3-Bromtoluol anstelle von Brombenzol eingesetzt werden. Man erhält 1,17 g Feststoff mit einer Reinheit laut GC/MS von 92%.
GC/MS: m/e = 217 (M⁺, 20%), 132 (M - NHCMe₂CO, 100%).

### Beispiel 4: 5,5-Dimethyl-3-(3-methylphenyl)pyrrolidin-2,4-dion

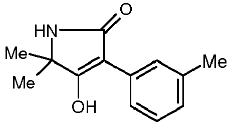

Unter Argon werden in einer ausgeheizten Apparatur 1,0 g festes Natriumhydroxid (in Form sogn. "Micropills") und 15 ml wasser- und luftfreies N-Methyl-pyrrolidon (NMP) vorgelegt. Unter Rühren gibt man dann 1,907 g 5,5-Dimethylpyrrolidin-2,4-dion hinzu und rührt 20 Minuten bei Raumtemperatur. Anschließend setzt man 1,71 g 3-Bromtoluol zu und erhitzt das Reaktionsgemisch auf 125°C. Bei dieser Temperatur gibt man dann 0,328 g Triphenylphosphin und 89 mg PdCl₂ hinzu. Man rührt 4 Stunden bei 125°C, lässt auf Raumtemperatur abkühlen, rührt das Reaktionsgemisch in 20 ml Eiswasser ein und stellt mit verdünnter Salzsäure auf pH 2. Man gibt 20 ml Methylenchlorid hinzu, verrührt, trennt die Phasen und schüttelt die wässrige Phase noch zweimal mit je 10 ml Methylenchlorid aus. Die vereinigten organischen Phasen werden getrocknet und dann am Rotationsverdampfer eingeengt. Es resultieren 1,82 g Zielprodukt (entsprechend einer Ausbeute von 84% der Theorie).

### Beispiel 5: 3-(4-Chlor-2-methylphenyl)-5,5-dimethylpyrrolidin-2,4-dion

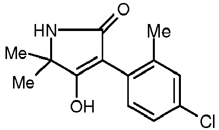

Man verfährt wie in Beispiel 1, mit dem Unterschied, dass 1,03 g 2-Brom-5-chlor-toluol anstelle von Brombenzol eingesetzt werden. Man erhält 1,37 g Feststoff mit einer Reinheit laut GC/MS von 94,3%.
GC/MS: m/e = 251 (M⁺für ³⁵Cl, 25%), 166 (M - NHCMe₂CO, 100%).

### Beispiel 6: 3-(Biphenyl-3-yl)-5,5-dimethylpyrrolidin-2,4-dion

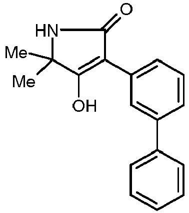

Man verfährt wie in Beispiel 1, mit dem Unterschied, dass 1,166 g 3-Brom-biphenyl anstelle von Brombenzol eingesetzt werden. Man erhält 1,52 g Feststoff mit einer Reinheit laut GC/MS von 95,4%.
GC/MS: m/e = 279 (M⁺, 35%), 194 (M - NHCMe₂CO, 90%), 165 (100%).

### Beispiel 7: 3-(2,5-Dimethylphenyl)-5,5-dimethylpyrrolidin-2,4-dion

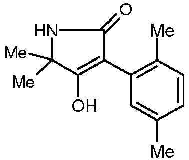

Man verfährt wie in Beispiel 1, mit dem Unterschied, dass 0,926 g 2,5-Dimethyl-brombenzol anstelle von Brombenzol eingesetzt werden. Man erhält 1,21 g Feststoff mit einer Reinheit laut GC/MS von 90%.
GC/MS: m/e = 231 (M⁺, 20%), 146 (M - NHCMe₂CO, 100%).

### Beispiel 8: 8-Methoxy-3-phenyl-1-azaspiro[4.5]decan-2,4-dion

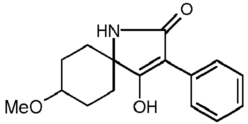

Man verfährt wie in Beispiel 1, mit dem Unterschied, dass 1,18 g 8-Methoxy-1-azaspiro[4.5]decan-2,4-dion anstelle von 5,5-Dimethylpyrrolidin-2,4-dion eingesetzt werden. Man erhält etwa 336 mg der Titelverbindung.
GC/MS: m/e = 273 (M⁺, 15%), 241 (M - MeOH, 5%), 118 (PhCHCO; 100%).

### Beispiel 9: 2,2-Dimethyl-5-oxo-4-phenyl-2,5-dihydro-1H-pyrrol-3-yl-ethylcarbonat

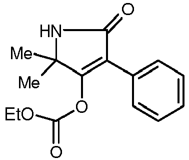

Man verfährt wie in Beispiel 1, mit dem Unterschied, dass 1,195 g 2,2-Dimethyl-5-oxo-2,5-dihydro-1H-pyrrol-3-yl-ethylcarbonat anstelle von 5,5-Dimethylpyrrolidin-2,4-dion eingesetzt werden. Man erhält die Titelverbindung in einer Ausbeute von 69% der Theorie.
GC/MS: m/e = 275 (M⁺, 2%), 203 (M-72, 80%), 188 (100%), 145 (95%), 118 (M - EtOCO, - NHCMe₂CO, 70%), 89 (100%).

### Beispiel 10: 2,2-Dimethyl-5-oxo-4-phenyl-2,5-dihydro-1H-pyrrol-3-ylacetat

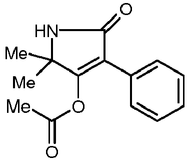

Man verfährt wie in Beispiel 1, mit dem Unterschied, dass 1,015 g 2,2-Dimethyl-5-oxo-2,5-dihydro-1H-pyrrol-3-ylacetat anstelle von 5,5-Dimethylpyrrolidin-2,4-dion eingesetzt werden. Man erhält die Titelverbindung in einer Ausbeute von etwa 35% der Theorie. Zusätzlich erhält man durch *in situ* Abspaltung des Acetylrestes 5,5-Dimethyl-3-phenylpyrrolidin-2,4-dion in einer Ausbeute von etwa 38% der Theorie.
GC/MS: m/e = 245 (M⁺, 2%), 203 (M-42, 100%), 188 (60%), 118 (80%), 43 (50%).

### Beispiel 11: 8-Methoxy-3-phenyl-1-azaspiro[4.5]decan-2,4-dion

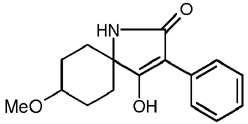

Man verfährt wie in Beispiel 1, mit dem Unterschied, dass 1,532 g Methyl-8-methoxy-2,4-dioxo-1-azaspiro[4.5]decan-3-carboxylat gemäß Beispiel (X-2-a-1) anstelle von 5,5-Dimethylpyrrolidin-2,4-dion eingesetzt werden. Man erhält die Titelverbindung in einer Ausbeute von etwa 90% der Theorie.

### Beispiel 12: 3-(4-Chlor-2-methylphenyl)-8-methoxy-1-azaspiro[4.5]decan-2,4-dion

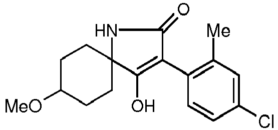

Man verfährt wie in Beispiel 11, mit dem Unterschied, dass 1,03 g 2-Brom-5-chlortoluol anstelle von Brombenzol eingesetzt werden. Man erhält die Titelverbindung in einer Ausbeute von etwa 22% der Theorie.
GC/MS: m/e = 321 (M⁺ für ³⁵Cl, 20%), 290 (M-31, 20%), 166 (100%).

### Beispiel 13: 3-(2,5-Dimethylphenyl)-8-methoxy-1-azaspiro[4.5]decan-2,4-dion

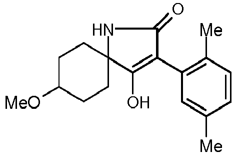

Man verfährt wie in Beispiel 11, mit dem Unterschied, dass 0,925 g 2,5-Dimethyl-brombenzol anstelle von Brombenzol eingesetzt werden. Man erhält die Titelverbindung in einer Ausbeute von etwa 20% der Theorie.
GC/MS: m/e = 301 (M⁺ 20%), 270 (M-31, 20%), 146 (100%).

### Beispiel 14: 3-[3-(4-Chlorphenyl)-6-methyl-phenyl]-5,5-dimethylpyrrolidin-2,4-dion

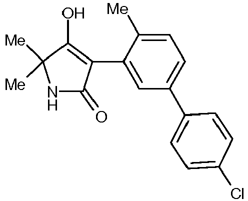

Unter Argon werden in einer ausgeheizten Apparatur 7,4 mg Pd(OAc)₂, 22 mg Di-tert-butyl(2'-methylbiphenyl-2-yl)phosphin und 0,78 g K₃PO₄ in 4,8 ml luftfreiem Dioxan vorgelet. Man gibt 203 mg 5,5-Dimethylpyrrolidin-2,4-dion und 659 mg 3-(4-Chlorphenyl)-6-methyl-brombenzol hinzu und rührt 16 Stunden unter Rückfluß. Danach lässt man auf Raumtemperatur abkühlen, verdünnt mit ca. 6 ml Methanol, filtriert und wäscht den Filterrückstand mit ca. 3 ml MeOH nach. Die vereinigten Filtrate werden am Rotationsverdampfer eingeengt. Der Rückstand wird in ca. 6 ml Wasser aufgenommen und mit 1 N Salzsäure schwach sauer gestellt. Der ausgefallene Feststoff wird abgesaugt und mit ca. 3 ml Wasser gewaschen. Dann wird er mit Aceton vom Filter gewaschen und das Filtrat eingeengt. Man erhält 0.597 g Feststoff. Nach Reversed-Phase-Trennung mit Wasser/Acetonitril (Gradient) erhält man 93 mg (14 % d. Theorie) mit einer Reinheit von 98,6 % nach HPLC/MS.
¹H-NMR (400 MHz, d₆-DMSO): δ = 1.36 (s, 6H, 2xCH₃), 2.20 (s, 3H, Ar-CH₃), 7.29-7.31 (d, 1H, ArH), 7.35 (d,1H, ArH), 7.47-7.51 (m, 3H, ArH), 7.61 (br, 1H, NH), 7.63-7.67 (m, 2H, ArH), 10.83 (s, br, 1H, OH) ppm.

### Beispiel (X-1-a-1)

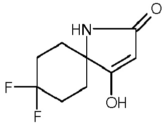

500 mg (1.9 mmol) der Verbindung gemäß Bsp. X-2-a-4 werden portionsweise innerhalb von 5 Minuten in ein siedendes 50%-iges Ethanol/Wassergemisch eingetragen. Man rührt unter Rückfluß bis die CO₂-Entwicklung beendet ist, rotiert ein und kristallisiert den Rückstand aus Ethanol um. Man erhält 275 mg eines farblosen Pulvers (69% d. Theorie).
¹H-NMR (400 MHz, CDCl₃): δ = 1.79-1.84 (m, 2H), 1.94 - 2.11 (2m, 4H), 2.20-2.33 (m, 2H), 3.13 (s, 2H, CO-CH₂-CO), 7.52 (br, 1H, NH) ppm.
¹H-NMR (400 MHz, CD₃CN): δ = 1.78-1.82 (m, 2H), 1.90 - 2.06 (2m, 4H), 2.12-2.18 (m, 2H), 3.03 (s, 2H, CO-CH₂-CO), 7.27 (br, 1H, NH) ppm.

### Beispiel (X-2-a-1)

### Verfahren B

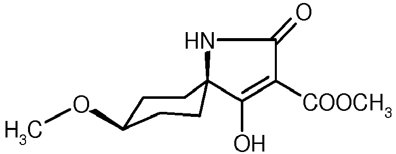

28.7 g (0.1 Mol) der Verbindung gemäß Bsp. XII-1 werden in 100 ml absoluten Methanol vorgelegt. Bei 20°C tropft man 19.5 ml Natriummmethylatlösung (30 %-ig in Methanol) zu und rührt 4 h bei 40°C nach. Das Lösungsmittel wird im Vakuum abgedampft , der Rückstand mit 50 ml Wasser aufgenommen und bei 0°C 110 ml 1 N Salzsäure zugetropft. Beim Eindampfen im Vakuum fällt das Produkt aus, wird anschließend in 50 ml Eiswasser suspendiert und abgesaugt. Ausbeute: 25 g (97 % d. Theorie) Fp. Zers.
¹H-NMR (400 MHz, d₆-DMSO): δ = 1.32-1.35 ("d", 2H), 1.39-1.49 (m, 2H), 1.65-1.73 (tm, 2H), 1.90-194 (dm, 2H), 3.09-3.16 (zm, 1H, CHOCH₃-cis), 3.24 (s, 3H, OCH₃), 3.59 (s, 3H, COOCH₃) ppm.
HPLC Retentionszeit 0.97 (Methode: Säule 50 x 4.6 mm Eclipse Plus C₁₈; 1.8 µm, Gradient 0.1% Phosphorsäure/Acetonitril; Fluss: 2 ml/min, 55 °C)

In Analogie zu Beispiel (X-2-a-1) und gemäß den allgemeinen Angaben zur Herstellung erhält man folgende Verbindungen der Formel (X-2-a):

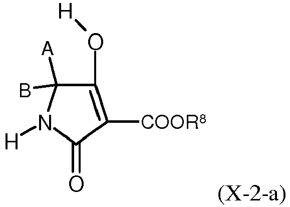

| **Bsp-Nr.** | **A** | **B** | **R⁸** | **Fp. °C; Analytik *** |
|---|---|---|---|---|
| X-2-a-2 | -(CH₂)₂-O-(CH₂)₂- | | CH₃ | 324; *¹ |
| X-2-a-3 | 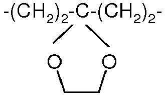 | | CH₃ | *² |
| X-2-a-4 | -(CH₂)₂-CF₂-(CH₂)₂- | | CH₃ | *³ |

| | | | | |
|---|---|---|---|---|
| *^{1 1}H-NMR (400 MHz, CD₃OD): δ = 1.43-1.47 (dd, 2H), 2.05-2.13 (tm, 2H), 3.66-3.72 (td, 2H, OCH₂), 3.80 (s, 3H, COOCH₃), 3.96-4.00 (d, m, 2H, OCH₂) ppm. *^{2 1}H-NMR (400 MHz, d₆-DMSO): δ = 1.17-1.2 (d, 2H), 1.56-1.86 (m, 6H), 3.46 (s, 3H, CO₂CH₃), 3.84 (s, 4H, -O(CH₂)₂-O), 7.23 (br, 1H, NH) ppm. *^{3 1}H-NMR (600 MHz, d₆-DMSO): δ = 1.52-1.54 (d, br, 2H), 1.91 (cm, br, 2H), 2.11-2.13 (2"d, br", 4H), 3.66 (s, 3H, CO₂CH₃), 8.85 (br, 1H, NH) ppm. | | | | |

### Beispiel XII-1

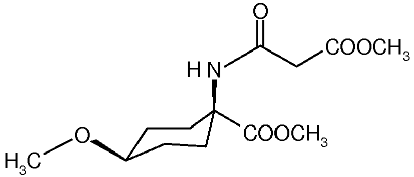

117.4 g (0.525 Mol) cis-1-Amino-4-methoxy-cyclohexancarbonsäure-methylester-hydrochlorid werden in 1000 ml absolutem Tetrahydrofuran (THF) vorgelegt, mit 153.3 ml (1.1 Mol) Triethylamin versetzt und bei 20°C 68.3 g (0.5 Mol) Malonsäuremethylesterchlorid in 30 ml absolutem THF zugetropft. Man rührt 4 h bei 40°C nach, gießt den Ansatz auf 1 1 Wasser, extrahiert mit Methylenchlorid, trocknet die organische Phase und dampft im Vakuum ein. Der Rückstand (172 g) wird durch Säulenchromatographie an Kieselgel mit Methylenchlorid/Essigsäureethylester 2:1 als Laufmittel gereinigt.
Ausbeute: 85.6 g (59.6 % d. Theorie), Fp. 74°C
¹H-NMR (400 MHz, CD₃CN): δ = 1.34-1.44 (qm, 2H), 1.73-1.81 (tm, 2H), 1.85-195 (m, 2H), 2.06-2.12 (dm, 2H), 3.15-3.22 (zm, 1H, CHOCH₃-cis), 3.24 (s, 2H, CH₂COOCH₃), 3.28 (s, 3H, OCH₃), 3.60, 3.68 (2s, je 3 H, COOCH₃), 6.88 (s, br, 1 H, NH) ppm.

In Analogie zu Beispiel (XII-1) und gemäß den allgemeinen Angaben zur Herstellung erhält man folgende Verbindungen der Formel (XII):

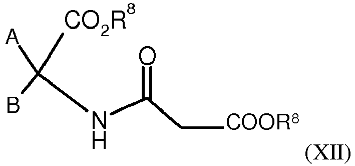

| **Bsp-Nr.** | **A** | **B** | **R⁸** | **Fp. °C; Analytik *** |
|---|---|---|---|---|
| XII-2 | -(CH₂)₂-O-(CH₂)₂- | | CH₃ | 62; *¹ |
| XII-3 | 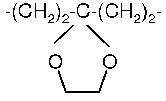 | | CH₃ | *² |
| XII-4 | -(CH₂)₂-CF₂-(CH₂)₂- | | CH₃ | *³ |

| | | | | |
|---|---|---|---|---|
| *^{1 1}H-NMR (400 MHz, CD₃CN): δ = 1.41-1.46 (dm, 1H), 1.86-2.08 (m, 3H), 3.25 (s, 2H, COCH2CO), 3.52-3.80 (m, 4H, OCH₂), 3.64, 3.68 (2s, je 3 H, COOMe), 7.06 (sbr, 1H, NH) ppm. *^{2 1}H-NMR (400 MHz, CDCl₃): δ = 1.67-1.76 (m, 4H), 2.14-2.19 (m, 4H), 3.34 (s, 2H, CO-CH₂-CO), 3.72, 3.77 (2s, je 3H, CO₂CH₃), 3.96 (s, 4H,-O-(CH₂)₂-O), 7.67 (s, br, 1H, NH) ppm. *^{3 1}H-NMR (400 MHz, d₆-DMSO): δ = 1.86-2.14 (m, 8H), 3.58 (s, 2H, COCH₂CO₂CH₃), 3.60, 3.63 (2s, je 3H, CO₂CH₃), 8.57 (s, br, 1H, NH) ppm. | | | | |

### Beispiel X-1-b-1: 2,2-Dimethyl-5-oxo-2,5-dihydro-1H-pyrrol-3-ylacetat

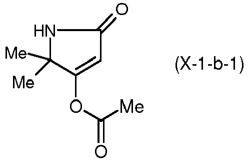

Zu einer Lösung von 6,36 g 5,5-Dimethylpyrrolidin-2,4-dion und 5,57 g Triethylamin in 50 ml Methylenchlorid tropft man bei 0 bis 5°C eine Lösung von 4,08 g Acetylchlorid in 20 Methylenchlorid. Man lässt innerhalb etwa einer Stunde auf Raumtemperatur kommen und rührt dann noch 24 Stunden. Anschließend wird das Reaktionsgemisch mit 50 ml Methylenchlorid verdünnt, zweimal mit je 50 ml Wasser, zweimal je 25 ml 5%iger Natronlauge und einmal 50 ml gesättigter wässriger NaCl-Lösung ausgeschüttelt. Nach Trocknen und Einengen erhält man 1,58 g der Titelverbindung in einer Reinheit n. HPLC von 97%.
LC/MS: m/e = 170 (MH⁺).
¹H-NMR (400 MHz, CDCl₃): δ = 1,33 (s, 6H), 2,23 (s, 3H), 5,91 (s, 1H), 7,05 (s,br, 1H) ppm.

### Beispiel X-1-c-1: 2,2-Dimethyl-5-oxo-2,5-dihydro-1H-pyrrol-3-yl-ethylcarbonat

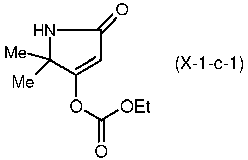

Zu einer Lösung von 6,36 g 5,5-Dimethylpyrrolidin-2,4-dion und 5,57 g Triethylamin in 50 ml Methylenchlorid tropft man bei 0 bis 5°C eine Lösung von 5,82 g Chlorameisensäure-ethylester in 20 Methylenchlorid. Man lässt innerhalb etwa einer Stunde auf Raumtemperatur kommen und rührt dann noch 24 Stunden. Anschließend wird das Reaktionsgemisch mit 50 ml Methylenchlorid verdünnt, zweimal mit je 50 ml Wasser, zweimal je 25 ml 5%iger Natronlauge und einmal 50 ml gesättigter wässriger NaCl-Lösung ausgeschüttelt. Nach Trocknen und Einengen erhält man 3,66 g der Titelverbindung in einer Reinheit n. HPLC von 98%.
LC/MS: m/e = 200 (MH⁺).
¹H-NMR (400 MHz, CDCl₃): δ = 1,31 - 1,35 (m, 9H), 4,25 - 4,30 (q, 2H), 5,88 (s, 1H), 7,29 (s,br, 1H) ppm.

### Beispiel XVIII-1:

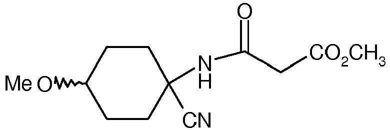

5.72 g (30 mmol) 1-Amino-4-methoxy-cyclohexancarbonsäurenitril hydrochlorid (cis/trans ca. 1:1) werden in 60 ml Tetrahydrofuran (THF) vorgelegt, mit 8.36 ml (60 mmol) Triethylamin und 10 mg Steglich-Base versetzt. Bei 0° - 10°C tropft man 4.1 g (30 mmol) Malonsäuremethylesterchlorid in 5 ml THF hinzu und rührt 4 h bei Raumtemperatur nach, saugt ab, wäscht mit THF nach und engt im Vakuum ein. Der Rückstand wird durch Flashsäulenchromatographie an Kieselgel mit Cyclohexan/Essigsäireethylester 2:1 gereinigt. Man erhält 4,96 g (65 % d. Theorie) eines cis/trans Isomerengemisches im Verhältnis von ca. 7:3.
¹H-NMR (400 MHz, d₆-DMSO): δ = 1,41-1.47 (m, 2H), 1.68-1.74 (m, 2H), 1.91-1.99 (m, 2H), 2.21-2.25 (m, 2H), 3.21, 3.24 (2s, trans/cis, zus. 3H, OCH₃), 3.22-3.27 (m, 1H, CHOCH₃), 3.32 (s, 2H, CH₂CO₂CH₃), 3.63 (s, 3H, CO₂CH₃), 8.56, 8.63 (2s, br, trans/cis, zus. 1H, NH) ppm.

### Beispiel XX-1:

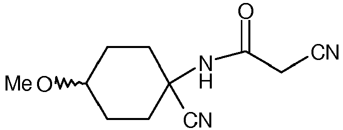

9.53 g (50 mmol) 1-Amino-4-methoxy-cyclohexancarbonsäurenitril x HCl (cis/trans Gemisch ca. 1:1) und 4.25 g (50 mmol) Cyanoessigsäure werden in 25 ml Pyridin vorgelegt. Anschließend tropft man ohne Kühlung 5.1 g (50 mmol) Acetonhydrid in 25 ml Pyridin zu und arbeitet nach der Zugabe sofort auf. Das Pyridin wird im Vakuum abgedampft, der Rückstand 2 x mit je 20 ml Toluol aufgenommen und wieder eingedampft. Anschließend wird Wasser zugegeben, mit Methylenchlorid extrahiert, getrocknet und eingedampft. Der Rückstand wird durch Flashchromatographie an Kieselgel mit Essigsäureetyhlester/Methanol Gradient 9:1 bis 4:1 vorgereinigt. Man erhält 6.86 g eines stark nach Essig riechenden Wachses, das aus 50 ml Essigsäureethylester umkristallisiert wird. Nach dem Absaugen erhält man 1.61 g (14.6 % d. Theorie) eines weißen Pulvers.
¹H-NMR (400 MHz, d₆-DMSO): δ = 1.37-1.46 (m, 2H), 1.67-1.72 (cm, 2H), 1.91-1.94 (m, 2H), 2.22-2.26 (m, 2H), 3.24 (s, 3H, OCH₃), 3.22-3.26 (m, 1H, CHOCH₃), 3,74 (s, 2H, CO-CH₂CN), 8.81 (s, br, 1H, NH) ppm.

## Patentansprüche

1. Verfahren zur Herstellung von Verbindungen der Formel (I) 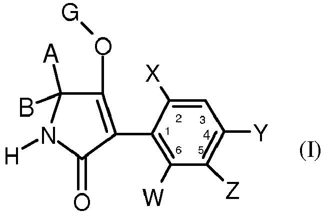 in welcher
W für Wasserstoff, Halogen, Alkyl, Alkenyl, Alkinyl, gegebenenfalls substituiertes Cycloalkyl, Alkoxy, Halogenalkyl oder Halogenalkoxy steht,
X für Wasserstoff, Halogen, Alkyl, Alkenyl, Alkinyl, gegebenenfalls substituiertes Cycloalkyl, Alkoxy, Halogenalkyl, Halogenalkoxy oder Cyano steht,
Y und Z unabhängig voneinander für Wasserstoff, Alkyl, Alkenyl, Alkinyl, Halogen, Cyano, gegebenenfalls substituiertes Cycloalkyl, Alkoxy, Halogenalkyl, Halogenalkoxy oder jeweils gegebenenfalls substituiertes Aryl oder Hetaryl stehen,
A für Wasserstoff, für jeweils gegebenenfalls durch Halogen substituiertes Alkyl, Alkenyl, Alkoxyalkyl, Alkylthioalkyl, gesättigtes oder ungesättigtes, gegebenenfalls substituiertes Cycloalkyl, in welchem gegebenenfalls mindestens ein Ringatom durch ein Heteroatom ersetzt ist, oder jeweils gegebenenfalls durch Halogen, Alkyl, Halogenalkyl, Alkoxy, Halogenalkoxy, Cyano oder Nitro substituiertes Aryl, Arylalkyl oder Hetaryl steht,
B für Wasserstoff, Alkyl oder Alkoxyalkyl steht, oder
A und B gemeinsam mit dem Kohlenstoffatom an das sie gebunden sind für einen gesättigten oder ungesättigten, gegebenenfalls mindestens ein Heteroatom enthaltenden unsubstituierten oder substituierten Cyclus stehen,
G für Wasserstoff (a) oder für eine der Gruppen 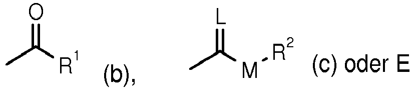
steht,
worin
E für ein Metallion oder ein Ammoniumion steht,
L für Sauerstoff oder Schwefel steht,
M für Sauerstoff oder Schwefel steht,
R¹ für jeweils gegebenenfalls durch Halogen oder Cyano substituiertes Alkyl, Alkenyl, Alkoxyalkyl, Alkylthioalkyl oder Polyalkoxyalkyl oder für jeweils gegebenenfalls durch Halogen, Alkyl oder Alkoxy substituiertes Cycloalkyl oder Heterocyclyl oder für jeweils gegebenenfalls substituiertes Phenyl, Phenylalkyl, Hetaryl, Phenoxyalkyl oder Hetaryloxyalkyl steht,
R² für jeweils gegebenenfalls durch Halogen oder Cyano substituiertes Alkyl, Alkenyl, Alkoxyalkyl oder Polyalkoxyalkyl oder für jeweils gegebenenfalls substituiertes Cycloalkyl, Phenyl oder Benzyl steht,
**dadurch gekennzeichnet, dass** man Verbindungen der Formel (X) 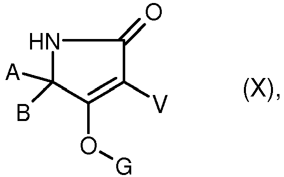 in welcher
A und B die oben angegebenen Bedeutungen haben,
G für die oben angegebenen Gruppen a), b), c) und E steht,
V für Wasserstoff steht oder
V für COOR⁸ steht,
wobei R⁸ für Alkyl steht
mit einer Verbindung der Formel (XI) 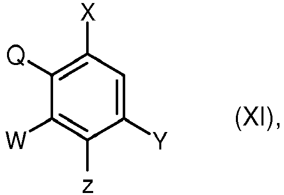 in welcher
W, X, Y und Z die oben angegebenen Bedeutungen haben mit der Ausnahme, dass als Halogen jetzt nur noch Fluor und Chlor stehen können, X auch zusätzlich für Wasserstoff stehen darf und
Q für Triflat, Brom oder Iod steht,
in Gegenwart einer Base, eines Palladiumkatalysators und eines Phosphinliganden der Formel (XII') 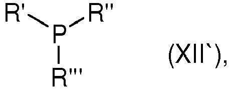 in welcher die Reste
R', R" und R'" unabhängig voneinander für C₁-C₁₂-Alkyl, C₅-C₁₀Cycloalkyl, C₆-C₁₀-Aryl, gegebenenfalls einfach oder mehrfach substituiert durch C₁-C₆-Alkyl, C₁-C₆-Alkoxy, C₁-C₆-Alkylamino, C₁-C₆-Dialkylamino oder gegebenenfalls einfach oder mehrfach durch C₁-C₆-Alkyl, C₁-C₆-Alkoxy, C₁-C₆-Alkylamino oder C₁-C₆-Dialkylamino substituiertes Phenyl stehen,
in einem Verdünnungsmittel umsetzt.

2. Verfahren zur Herstellung von Verbindungen der Formel (I) gemäß Anspruch 1, in welcher
W für Wasserstoff, Chlor, Brom, C₁-C₄-Alkyl, C₂-C₄-Alkenyl, C₂-C₄-Alkinyl, gegebenenfalls einfach durch Methyl, Ethyl, Methoxy, Fluor, Chlor, Trifluormethyl oder Cyclopropyl substituiertes C₃-C₆-Cycloalkyl, C₁-C₄-Alkoxy, C₁-C₂-Halogenalkyl oder C₁-C₂-Halogenalkoxy steht,
X für Wasserstoff, Chlor, Brom, Iod, C₁-C₄-Alkyl, C₂-C₄-Alkenyl, C₂-C₄-Alkinyl, gegebenenfalls einfach durch Methyl, Ethyl, Methoxy, Fluor, Chlor, Trifluormethyl oder Cyclopropyl substituiertes C₃-C₆-Cycloalkyl, C₁-C₄-Alkoxy, C₁-C₄-Halogenalkyl, C₁-C₄-Halogenalkoxy oder Cyano steht,
Y und Z unabhängig voneinander für Wasserstoff, Cyano, Fluor, Chlor, Brom, Iod, C₁-C₄-Alkyl, C₂-C₄-Alkenyl, C₂-C₄-Alkinyl, gegebenenfalls einfach durch Methyl, Ethyl, Methoxy, Fluor, Chlor, Trifluormethyl oder Cyclopropyl substituiertes C₃-C₆-Cycloalkyl, C₁-C₆-Alkoxy, C₁-C₄-Halogenalkyl, C₁-C₄-Halogenalkoxy, oder für einen der (Het)-arylreste stehen, 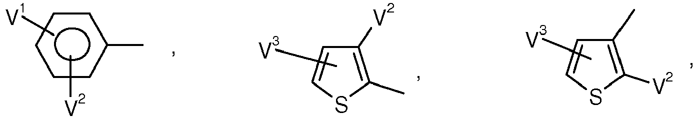 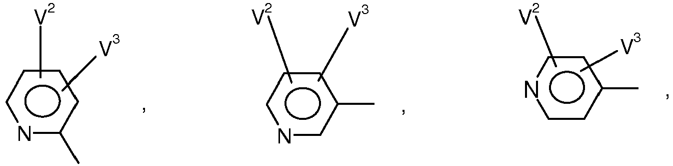 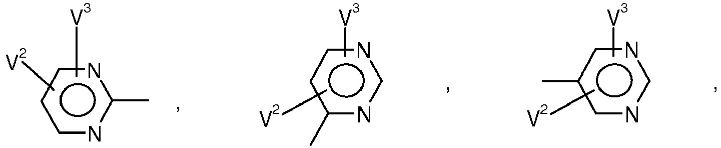
wobei im Falle von (Het)-aryl nur einer der Reste Y oder Z für (Het)-aryl stehen darf,
V¹ für Wasserstoff, Fluor, Chlor, Brom, C₁-C₆-Alkyl, C₁-C₄-Alkoxy, C₁-C₂-Halogenalkyl, C₁-C₂-Halogenalkoxy, Nitro, Cyano oder gegebenenfalls einfach bis zweifach durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenyl steht,
V² und V³ unabhängig voneinander für Wasserstoff, Fluor, Chlor, Brom, C₁-C₄-Alkyl, C₁-C₄-Alkoxy, C₁-C₂-Halogenalkyl oder C₁-C₂-Halogenalkoxy stehen,
A für Wasserstoff, jeweils gegebenenfalls einfach bis dreifach durch Fluor oder Chlor substituiertes C₁-C₆-Alkyl, C₁-C₄-Alkoxy-C₁-C₂-alkyl, gegebenenfalls einfach bis zweifach durch C₁-C₂-Alkyl oder C₁-C₂-Alkoxy substituiertes und gegebenenfalls durch ein Sauerstoffatom unterbrochenes C₃-C₆-Cycloalkyl oder jeweils gegebenenfalls einfach bis zweifach durch Fluor, Chlor, Brom, C₁-C₄-Al-kyl, C₁-C₂-Halogenalkyl, C₁-C₄-Alkoxy, C₁-C₂-Halogenalkoxy, Cyano oder Nitro substituiertes Phenyl, Pyridyl oder Benzyl steht,
B für Wasserstoff, C₁-C₄-Alkyl oder C₁-C₂-Alkoxyl-C₁-C₂-alkyl steht oder
A, B und das Kohlenstoffatom an das sie gebunden sind, für gesättigtes oder ungesättigtes C₃-C₇-Cycloalkyl stehen, worin gegebenenfalls ein Ringglied durch Stickstoff, Sauerstoff oder Schwefel ersetzt ist und welches gegebenenfalls einfach bis zweifach durch Halogen, C₁-C₆-Alkyl, C₁-C₄-Alkoxy-C₁-C₂-alkyl, Trifluormethyl, C₁-C₆-Alkoxy, C₃-C₆-Alkenyloxy, Trifluorethoxy, C₁-C₃-Alkoxy-C₁-C₃-alkoxy oder C₃-C₆-Cycloalkylmethoxy substituiert ist, wobei die zuvor genannten Reste (ausgenommen Halogen und Trifluormethyl) auch als N-Substituenten in Frage kommen, oder
A, B und das Kohlenstoffatom, an das sie gebunden sind, für C₅-C₆-Cycloalkyl stehen, welches durch eine gegebenenfalls mit ein oder zwei nicht direkt benachbarte Sauerstoff- oder Schwefelatome enthaltende gegebenenfalls durch Methyl oder Ethyl substituierte Alkylendiyl- oder durch eine Alkylendioxyl- oder durch eine Alkylendithiol-Gruppe substituiert ist, die mit dem Kohlenstoffatom, an das sie gebunden ist, einen weiteren fünf- oder sechsgliedrigen Ring bildet, oder
A, B und das Kohlenstoffatom, an das sie gebunden sind, für C₃-C₆-Cycloalkyl oder C₅-C₆-Cycloalkenyl stehen, in welchen zwei Substituenten gemeinsam mit den Kohlenstoffatomen, an die sie gebunden sind, für jeweils gegebenenfalls durch C₁-C₂-Alkyl oder C₁-C₂-Alkoxy substituiertes C₂-C₄-Alkandiyl, C₂-C₄-Alkendiyl oder Butadiendiyl stehen,
G für Wasserstoff (a) oder für eine der Gruppen 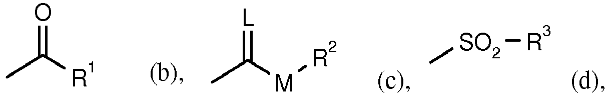 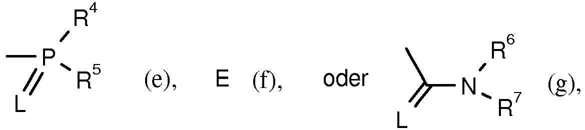 steht
in welchen
E für ein Metallion oder ein Ammoniumion steht,
L für Sauerstoff oder Schwefel steht und
M für Sauerstoff oder Schwefel steht,
R¹ für jeweils gegebenenfalls einfach bis dreifach durch Fluor oder Chlor substituiertes C₁-C₁₆-Alkyl, C₂-C₁₆-Alkenyl, C₁-C₆-Alkoxy-C₁-C₄-alkyl, C₁-C₆-Alkylthio-C₁-C₄-alkyl oder Poly-C₁-C₆-alkoxy-C₁-C₄-alkyl oder für gegebenenfalls einfach bis zweifach durch Fluor, Chlor, C₁-C₅-Alkyl oder C₁-C₅-Alkoxy substituiertes C₃-C₇-Cycloalkyl, in welchem gegebenenfalls eine oder zwei nicht direkt benachbarte Methylengruppen durch Sauerstoff und/oder Schwefel ersetzt sind,
für gegebenenfalls einfach bis dreifach durch Fluor, Chlor, Brom, Cyano, Nitro, C₁-C₄-Alkyl, C₁-C₄-Alkoxy, C₁-C₃-Halogenalkyl, C₁-C₃-Halogenalkoxy, C₁-C₄-Alkylthio oder C₁-C₄-Alkylsulfonyl substituiertes Phenyl,
für gegebenenfalls einfach bis zweifach durch Fluor, Chlor, Brom, C₁-C₄-Alkyl, C₁-C₄-Alkoxy, C₁-C₃-Halogenalkyl oder C₁-C₃-Halogenalkoxy substituiertes Phenyl-C₁-C₄-alkyl,
für jeweils gegebenenfalls einfach bis zweifach durch Fluor, Chlor, Brom oder C₁-C₄-Alkyl substituiertes Pyrazolyl, Thiazolyl, Pyridyl, Pyrimidyl, Furanyl oder Thienyl,
für gegebenenfalls einfach bis zweifach durch Fluor, Chlor, Brom oder C₁-C₄-Alkyl substituiertes Phenoxy-C₁-C₅-alkyl oder
für jeweils gegebenenfalls einfach bis zweifach durch Fluor, Chlor, Brom, Amino oder C₁-C₄-Alkyl substituiertes Pyridyloxy-C₁-C₅-alkyl, Pyrimidyloxy-C₁-C₅-alkyl oder Thiazolyloxy-C₁-C₅-alkyl steht,
R² für jeweils gegebenenfalls einfach bis dreifach durch Fluor oder Chlor substituiertes C₁-C₁₆-Alkyl, C₂-C₁₆-Alkenyl, C₁-C₆-Alkoxy-C₂-C₆-alkyl oder Poly-C₁-C₆-alkoxy-C₂-C₆-alkyl,
für gegebenenfalls einfach bis zweifach durch Fluor, Chlor, C₁-C₄-Alkyl oder C₁-C₄-Alkoxy substituiertes C₃-C₇-Cycloalkyl oder
für jeweils gegebenenfalls einfach bis dreifach durch Fluor, Chlor, Brom, Cyano, Nitro, C₁-C₄-Alkyl, C₁-C₃-Alkoxy, C₁-C₃-Halogenalkyl oder C₁-C₃-Halogenalkoxy substituiertes Phenyl oder Benzyl steht,
R³ für gegebenenfalls einfach bis dreifach durch Fluor oder Chlor substituiertes C₁-C₆-Alkyl oder jeweils gegebenenfalls einfach bis zweifach durch Fluor, Chlor, Brom, C₁-C₄-Alkyl, C₁-C₄-Alkoxy, C₁-C₂-Halogenalkoxy, C₁-C₂-Halogenalkyl, Cyano oder Nitro substituiertes Phenyl oder Benzyl steht,
R⁴ und R⁵ unabhängig voneinander für jeweils gegebenenfalls einfach bis dreifach durch Fluor oder Chlor substituiertes C₁-C₆-Alkyl, C₁-C₆-Alkoxy, C₁-C₆-Alkylamino, Di-(C₁-C₆-alkyl)amino, C₁-C₆-Alkylthio oder C₃-C₄-Alkenylthio oder für jeweils gegebenenfalls einfach bis zweifach durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl, oder C₁-C₃-Halogenalkyl substituiertes Phenyl, Phenoxy oder Phenylthio stehen,
R⁶ und R⁷ unabhängig voneinander für Wasserstoff, für jeweils gegebenenfalls einfach bis dreifach durch Fluor oder Chlor substituiertes C₁-C₆-Alkyl, C₃-C₆-Cycloalkyl, C₁-C₆-Alkoxy, C₃-C₆-Alkenyl oder C₁-C₆-Alkoxy-C₂-C₆-alkyl, für jeweils gegebenenfalls einfach bis dreifach durch Fluor, Chlor, Brom, C₁-C₅-Halogenalkyl, C₁-C₅-Alkyl oder C₁-C₅-Alkoxy substituiertes Phenyl oder Benzyl, oder zusammen für einen gegebenenfalls durch C₁-C₄-Alkyl substituierten C₃-C₆-Alkylenrest stehen, in welchem gegebenenfalls eine Methylengruppe durch Sauerstoff oder Schwefel ersetzt ist.

3. Verfahren zur Herstellung von Verbindungen der Formel (I) gemäß Anspruch 1, in welcher
W für Wasserstoff, Chlor, Brom, Methyl, Ethyl, Methoxy, Ethoxy oder Trifluormethyl steht,
X für Wasserstoff, Chlor, Brom, Jod, Methyl, Ethyl, Propyl, Cyclopropyl, Methoxy, Ethoxy, Trifluormethyl oder Trifluormethoxy steht,
Y und Z unabhängig voneinander für Wasserstoff, Fluor, Chlor, Brom, Iod, Methyl, Ethyl, Cyclopropyl, Methoxy, Trifluormethyl, Trifluormethoxy oder einen Phenylrest, 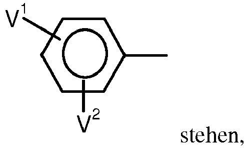
wobei im Falle von Phenyl nur einer der Reste Y oder Z für Phenyl stehen darf,
V¹ für Wasserstoff, Fluor oder Chlor steht,
V² für Wasserstoff, Fluor, Chlor, Methyl, Ethyl, n-Propyl, iso-Propyl, Methoxy, Ethoxy oder Trifluormethyl steht,
A für Wasserstoff, jeweils gegebenenfalls einfach bis dreifach durch Fluor substituiertes C₁-C₄-Alkyl oder C₁-C₂-Alkoxy-C₁-C₂-alkyl, für Cyclopropyl, Cyclopentyl oder Cyclohexyl steht,
B für Wasserstoff, Methyl oder Ethyl steht oder
A, B und das Kohlenstoffatom an das sie gebunden sind, für gesättigtes C₅-C₆-Cycloalkyl stehen, in welchem gegebenenfalls ein Ringglied durch Stickstoff, Sauerstoff oder Schwefel ersetzt ist und welches gegebenenfalls einfach oder zweifach durch Fluor, Chlor, Methyl, Ethyl, Methoxymethyl, Ethoxymethyl, Methoxyethyl, Ethoxyethyl, Trifluormethyl, Methoxy, Ethoxy, Propoxy, Butoxy, Methoxyethoxy, Ethoxyethoxy, Allyloxy, Trifluorethoxy oder Cyclopropylmethoxy substituiert ist, wobei die zuvor genannten Reste (ausgenommen Fluor, Chlor, Trifluormethyl) auch als N-Substituenten in Frage kommen,
A, B und das Kohlenstoffatom, an das sie gebunden sind, für C₆-Cycloalkyl stehen, welches gegebenenfalls durch eine gegebenenfalls durch eine mit einem Sauerstoffatom unterbrochene Alkylidendiyl-Gruppe oder durch eine mit zwei nicht direkt benachbarten Sauerstoffatomen enthaltende Alkylidendiyl-Gruppe substituiert ist, wobei ein 5- oder 6-Ringketal gebildet wird und die jeweils gegebenenfalls einfach oder zweifach durch Methyl subsitutiert sein können, oder
A, B und das Kohlenstoffatom, an das sie gebunden sind, für C₅-C₆-Cycloalkyl oder C₅-C₆-Cycloalkenyl stehen, worin zwei Substituenten gemeinsam mit den Kohlenstoffatomen, an die sie gebunden sind, für C₂-C₄-Alkandiyl oder C₂-C₄-Alkendiyl oder Butadiendiyl stehen,
G für Wasserstoff (a) oder für eine der Gruppen 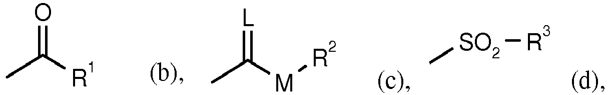 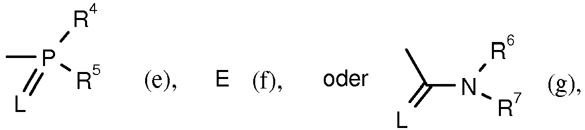 steht,
in welchen
E für ein Metallion oder ein Ammoniumion steht,
L für Sauerstoff oder Schwefel steht und
M für Sauerstoff oder Schwefel steht,
R¹ für jeweils gegebenenfalls einfach bis dreifach durch Fluor oder Chlor substituiertes C₁-C₁₀-Alkyl, C₂-C₁₀-Alkenyl, C₁-C₄-Alkoxy-C₁-C₂-alkyl, C₁-C₄-Alkylthio-C₁-C₂-alkyl oder für gegebenenfalls einfach durch Fluor, Chlor, Methyl, Ethyl oder Methoxy substituiertes C₃-C₆-Cycloalkyl, für gegebenenfalls einfach bis zweifach durch Fluor, Chlor, Brom, Cyano, Nitro, Methyl, Ethyl, n-Propyl, i-Propyl, Methoxy, Ethoxy, Trifluormethyl oder Trifluormethoxy substituiertes Phenyl, für jeweils gegebenenfalls einfach durch Chlor, Brom oder Methyl substituiertes Furanyl, Thienyl oder Pyridyl steht,
R² für jeweils gegebenenfalls einfach bis dreifach durch Fluor oder Chlor substituiertes C₁-C₁₀-Alkyl, C₂-C₁₀-Alkenyl oder C₁-C₄-Alkoxy-C₂-C₄-alkyl, für Cyclopentyl oder Cyclohexyl oder für jeweils gegebenenfalls einfach bis zweifach durch Fluor, Chlor, Cyano, Nitro, Methyl, Ethyl, Methoxy, Trifluormethyl oder Trifluormethoxy substituiertes Phenyl oder Benzyl steht,
R³ für jeweils gegebenenfalls einfach bis dreifach durch Fluor oder Chlor substituiertes Methyl, Ethyl, Propyl oder iso-Propyl, oder jeweils gegebenenfalls einfach durch Fluor, Chlor, Brom, Methyl, Ethyl, iso-Propyl, tert.-Butyl, Methoxy, Ethoxy, iso-Propoxy, Trifluormethyl, Trifluormethoxy, Cyano oder Nitro substituiertes Phenyl steht,
R⁴ und R⁵ unabhängig voneinander für C₁-C₄-Alkoxy oder C₁-C₄-Alkylthio oder für jeweils gegebenenfalls einfach durch Fluor, Chlor, Brom, Nitro, Cyano, Methyl, Methoxy, Trifluormethyl oder Trifluormethoxy substituiertes Phenyl, Phenoxy oder Phenylthio stehen,
R⁶ und R⁷ unabhängig voneinander für Wasserstoff, für C₁-C₄-Alkyl, C₃-C₆-Cycloalkyl, C₁-C₄-Alkoxy, C₃-C₄-Alkenyl oder C₁-C₄-Alkoxy-C₂-C₄-alkyl, für gegebenenfalls einfach bis zweifach durch Fluor, Chlor, Brom, Methyl, Methoxy oder Trifluormethyl substituiertes Phenyl, oder zusammen für einen C₅-C₆-Alky-lenrest stehen, in welchem gegebenenfalls eine Methylengruppe durch Sauerstoff oder Schwefel ersetzt ist.

4. Verfahren zur Herstellung von Verbindungen der Formel (I) gemäß Anspruch 1, in welcher
W für Wasserstoff, Chlor, Methyl, Ethyl oder Methoxy steht,
X für Wasserstoff, Chlor, Methyl, Ethyl, Methoxy oder Ethoxy steht,
Y und Z unabhängig voneinander für Wasserstoff, Chlor, Methyl oder für den Rest 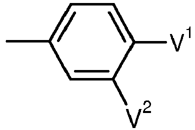 stehen, wobei in diesem Falle nur einer der Reste Y oder Z für 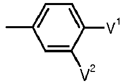 stehen darf,
V¹ für Wassersoff, Fluor oder Chlor steht,
V² für Wasserstoff, Fluor oder Chlor steht,
A für Methyl, Ethyl, Propyl, iso-Propyl oder Cyclopropyl steht,
B für Wasserstoff oder Methyl steht,
A, B und das Kohlenstoffatom an das sie gebunden sind, für gesättigtes C₅-C₆-Cycloalkyl stehen, in welchem gegebenenfalls ein Ringglied durch Sauerstoff ersetzt ist und welches gegebenenfalls einfach durch Fluor, Chlor, Methyl, Ethyl, Methoxymethyl, Methoxy, Ethoxy, Propoxy, Butoxy, Trifluorethoxy substituiert ist, oder
A, B und das Kohlenstoffatom, an das sie gebunden sind, für C₆-Cycloalkyl stehen, welches gegebenenfalls durch eine gegebenenfalls durch eine mit Sauerstoff unterbrochene Alkylidendiyl-Gruppe oder durch eine mit zwei nicht direkt benachbarten Sauerstoffatomen enthaltende Alkylidendiyl-Gruppe substituiert ist, wobei ein 5- oder 6-Ringketal gebildet wird, die jeweils einfach oder zweifach durch Methyl substituiert sein können,
G für Wasserstoff (a) oder für eine der Gruppen 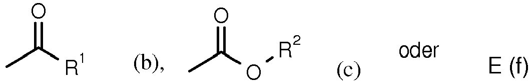 steht,
in welchen
E für ein Lithium-, Natrium-, Kalium-, Rubidium-, Cäsium-, Magnesium-, Calciumion oder ein Ammoniumion steht,
R¹ für Methyl, Ethyl, Propyl, iso-Propyl, Butyl, iso-Butyl, sek.-Butyl, tert.-Butyl, Cyclopropyl, Cyclopentyl oder Cyclohexyl steht,
R² für Methyl, Ethyl, Propyl, iso-Propyl, Butyl, iso-Butyl, sek.-Butyl, tert.-Butyl, oder für Benzyl steht.

## Claims

1. Process for the preparation of compounds of the formula (I) 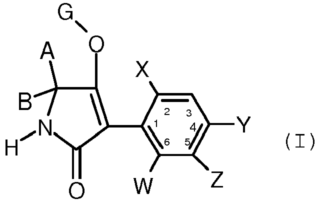 in which
W is hydrogen, halogen, alkyl, alkenyl, alkynyl, optionally substituted cyclocalkyl, alkoxy, haloalkyl or haloalkoxy,
X is hydrogen, halogen, alkyl, alkenyl, alkynyl, optionally substituted cycloalkyl, alkoxy, haloalkyl, haloalkoxy or cyano,
Y and Z, independently of one another, are hydrogen, alkyl, alkenyl, alkynyl, halogen, cyano, optionally substituted cycloalkyl, alkoxy, haloalkyl, haloalkoxy or in each case hoptionally substituted aryl or hetaryl,
A is hydrogen, is in each case optionally halogen-substituted alkyl, alkenyl, alkoxyalkyl, alkylthioalkyl, saturated or unsaturated, optionally substituted cycloalkyl, in which optionally at least one ring atom is replaced by a heteroatom, or aryl, arylalkyl or hetaryl each of which is optionally substituted by halogen, alkyl, haloalkyl, alkoxy, haloalkoxy, cyano or nitro,
B is hydrogen, alkyl or alkoxyalkyl, or
A and B, together with the carbon atom to which they are bonded are a saturated or unsaturated unsubstituted or substituted cycle optionally containing at least one heteroatom,
G is hydrogen (a) or is one of the groups 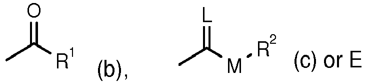
in which
E is a metal ion or an ammonium ion,
L is oxygen or sulphur,
M is oxygen or sulphur,
R¹ is alkyl, alkenyl, alkoxyalkyl, alkylthioalkyl or polyalkoxyalkyl each of which is optionally substituted by halogen or cyano, or is cycloalkyl or heterocyclyl each of which is optionally substituted by halogen, alkyl or alkoxy, or is in each case optionally substituted phenyl, phenylalkyl, hetaryl, phenoxyalkyl or hetaryloxyalkyl,
R² is alkyl, alkenyl, alkoxyalkyl or polyalkoxyalkyl each of which is optionally substituted by halogen or cyano, or is in each case optionally substituted cycloalkyl, phenyl or benzyl,
**characterized in that** compounds of the formula (X) 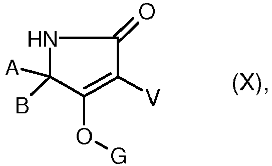 in which
A and B have the meanings given above,
G is the groups a), b), c) and E given above, V is hydrogen or
V is COOR⁸,
where R⁸ is alkyl
are reacted with a compound of the formula (XI) 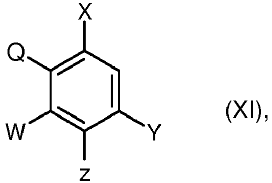
in which
W, X, Y and Z have the meanings given above, with the exception that the halogen can now only be fluorine and chlorine, X may also additionally be hydrogen and
Q is triflate, bromine or iodine,
in the presence of a base, a palladium catalyst and a phosphine ligand of the formula (XII') 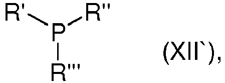 in which the radicals
R', R" and R''' independently of one another are C₁-C₁₂-alkyl, C₅-C₁₀-cycloalkyl, C₆-C₁₀-aryl, optionally mono- or polysubstituted by C₁-C₆-alkyl, C₁-C₆-alkoxy, C₁-C₆-alkylamino, C₁-C₆-dialkylamino or phenyl optionally mono- or polysubstituted by C₁-C₆-alkyl, C₁-C₆-alkoxy, C₁-C₆-alkylamino or C₁-C₆-dialkylamino,
in a diluent.

2. Process for the preparation of compounds of the formula (I) according to Claim 1, in which
W is hydrogen, chlorine, bromine, C₁-C₄-alkyl, C₂-C₄-alkenyl, C₂-C₄-alkynyl, C₃-C₆-cycloalkyl optionally monosubstituted by methyl, ethyl, methoxy, fluorine, chlorine, trifluoromethyl or cyclopropyl, C₁-C₄-alkoxy, C₁-C₂-haloalkyl or C₁-C₂-haloalkoxy,
X is hydrogen, chlorine, bromine, iodine, C₁-C₄-alkyl, C₂-C₄-alkenyl, C₂-C₄-alkynyl, C₃-C₆-cycloalkyl optionally monosubstituted by methyl, ethyl, methoxy, fluorine, chlorine, trifluoromethyl or cyclopropyl, C₁-C₄-alkoxy, C₁-C₄-haloalkyl, C₁-C₄-haloalkoxy or cyano,
Y and Z are, independently of one another, hydrogen, cyano, fluorine, chlorine, bromine, iodine, C₁-C₄-alkyl, C₂-C₄-alkenyl, C₂-C₄-alkynyl, C₃-C₆-cycloalkyl optionally monosubstituted by methyl, ethyl, methoxy, fluorine, chlorine, trifluoromethyl or cyclopropyl, C₁-C₆-alkoxy, C₁-C₄-haloalkyl, C₁-C₄-haloalkoxy, or is one of the (Het)-aryl radicals, 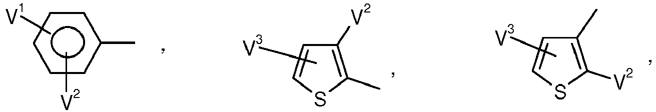 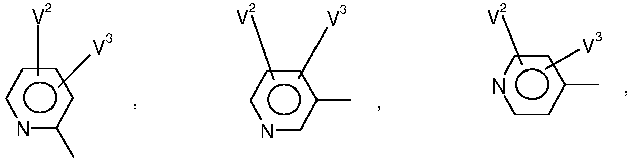 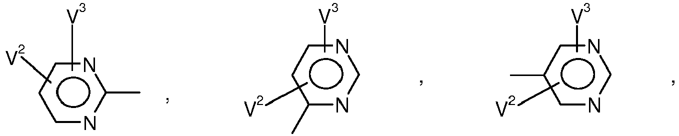
where in the case of (Het)-aryl only one of the radicals Y or Z may be (Het)-aryl,
V¹ is hydrogen, fluorine, chlorine, bromine, C₁-C₆-alkyl, C₁-C₄-alkoxy, C₁-C₂- haloalkyl, C₁-C₂-halo-alkoxy, nitro, cyano or phenyl optionally mono- to disubstituted by fluorine, chlorine, bromine, C₁-C₄-alkyl, C₁-C₄-alkoxy, C₁-C₂-haloalkyl, C₁-C₂-haloalkoxy, nitro or cyano,
V² and V³ are, independently of one another, hydrogen, fluorine, chlorine, bromine, C₁-C₄-alkyl, C₁-C₄-alkoxy, C₁-C₂-haloalkyl or C₁-C₂-haloalkoxy,
A is hydrogen, C₁-C₆-alkyl, C₁-C₄-alkoxy-C₁-C₂-alkyl, each of which is optionally mono- to trisubstituted by fluorine or chlorine, C₃-C₆-cycloalkyl which is optionally mono- to disubstituted by C₁-C₂-alkyl or C₁-C₂-alkoxy and which is optionally interrupted by an oxygen atom, or phenyl, pyridyl or benzyl each of which is optionally mono- to disubstituted by fluorine, chlorine, bromine, C₁-C₄-alkyl, C₁-C₂-haloalkyl, C₁-C₄-alkoxy, C₁-C₂-haloalkoxy, cyano or nitro,
B is hydrogen, C₁-C₄-alkyl or C₁-C₂-alkoxy-C₁-C₂-alkyl or
A, B and the carbon atom to which they are bonded are saturated or unsaturated C₃-C₇-cycloalkyl in which optionally one ring member is replaced by nitrogen, oxygen or sulphur and which is optionally mono- to disubstituted by halogen, C₁-C₆-alkyl, C₁-C₄-alkoxy-C₁-C₂-alkyl, trifluoromethyl, C₁-C₆-alkoxy, C₃-C₆-alkenyloxy, trifluoroethoxy, C₁-C₃-alkoxy-C₁-C₃-alkoxy or C₃-C₆-cycloalkylmethoxy, where the aforementioned radicals (with the exception of halogen and trifluoromethyl) are also suitable as N substituents, or
A, B and the carbon atom to which they are bonded are C₅-C₆-cycloalkyl which is substituted by an alkylenediyl group or by an alkylenedioxyl group or by an alkylenedithiol group which optionally contains with one or two non-directly adjacent oxygen or sulphur atoms and which is optionally substituted by methyl or ethyl, and which, with the carbon atom to which it is bonded, forms a further five- or six-membered ring, or
A, B and the carbon atom to which they are bonded are C₃-C₆-cycloalkyl or C₅-C₆-cycloalkenyl in which two substituents, together with the carbon atoms to which they are bonded, are C₂-C₄-alkanediyl, C₂-C₄-alkenediyl or butadienediyl each of which is optionally substituted by C₁-C₂-alkyl or C₁-C₂-alkoxy,
G is hydrogen (a) or is one of the groups 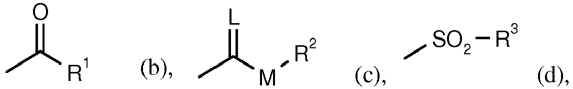 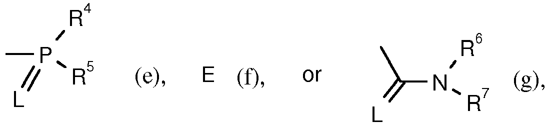
in which
E is a metal ion or an ammonium ion,
L is oxygen or sulphur and
M is oxygen or sulphur,
R¹ is C₁-C₁₆-alkyl, C₂-C₁₆-alkenyl, C₁-C₆-alkoxy-C₁-C₄-alkyl, C₁-C₆-alkylthio-C₁-C₄-alkyl or poly-C₁-C₆-alkoxy-C₁-C₄-alkyl each of which is optionally mono- to trisubstituted by fluorine or chlorine, or is C₃-C₇-cycloalkyl optionally mono- to disubstituted by fluorine, chlorine, C₁-C₅-alkyl or C₁-C₅-alkoxy, in which optionally one or two non-directly adjacent methylene groups are replaced by oxygen and/or sulphur,
is phenyl optionally mono- to trisubstituted by fluorine, chlorine, bromine, cyano, nitro, C₁-C₄-alkyl, C₁-C₄-alkoxy, C₁-C₃-haloalkyl, C₁-C₃-halo-alkoxy, C₁-C₄-alkylthio or C₁-C₄-alkylsulphonyl,
is phenyl C₁-C₄-alkyl optionally mono- to disubstituted by fluorine, chlorine, bromine, C₁-C₄-alkyl, C₁-C₄-alkoxy, C₁-C₃-haloalkyl or C₁-C₃-haloalkoxy,
is pyrazolyl, thiazolyl, pyridyl, pyrimidyl, furanyl or thienyl each of which is optionally mono-to disubstituted by fluorine, chlorine, bromine or C₁-C₄-alkyl,
is phenoxy-C₁-C₅-alkyl optionally mono- to disubstituted by fluorine, chlorine, bromine or C₁-C₄-alkyl, or
is pyridyloxy-C₁-C₅-alkyl, pyrimidyloxy-C₁-C₅-alkyl or thiazolyloxy-C₁-C₅-alkyl each of which is optionally mono- to disubstituted by fluorine, chlorine, bromine, amino or C₁-C₄-alkyl,
R² is C₁-C₁₆-alkyl, C₂-C₁₆-alkenyl, C₁-C₆-alkoxy-C₂-C₆-alkyl or poly-C₁-C₆-alkoxy-C₂-C₆-alkyl each of which is optionally mono- to trisubstituted by fluorine or chlorine,
is C₃-C₇-cycloalkyl optionally mono- to disubstituted by fluorine, chlorine, C₁-C₄-alkyl or C₁-C₄-alkoxy or
is phenyl or benzyl each of which is optionally mono- to trisubstituted by fluorine, chlorine, bromine, cyano, nitro, C₁-C₄-alkyl, C₁-C₃-alkoxy, C₁-C₃-haloalkyl or C₁-C₃-haloalkoxy,
R³ is C₁-C₆-alkyl optionally mono- to trisubstituted by fluorine or chlorine, or phenyl or benzyl each of which is optionally mono- to disubstituted by fluorine, chlorine, bromine, C₁-C₄-alkyl, C₁-C₄-alkoxy, C₁-C₂-haloalkoxy, C₁-C₂-haloalkyl, cyano or nitro,
R⁴ and R⁵, independently of one another, are C₁-C₆-alkyl, C₁-C₆-alkoxy, C₁-C₆-alkylamino, di(C₁-C₆-alkyl)amino, C₁-C₆-alkylthio or C₃-C₄-alkenylthio each of which is optionally mono- to trisubstituted by fluorine or chlorine, or are phenyl, phenoxy or phenylthio each of which is optionally mono- to disubstituted by fluorine, chlorine, bromine, nitro, cyano, C₁-C₃-alkoxy, C₁-C₃-haloalkoxy, C₁-C₃-alkylthio, C₁-C₃-halo-alkylthio, C₁-C₃-alkyl, or C₁-C₃-haloalkyl,
R⁶ and R⁷, independently of one another, are hydrogen, C₁-C₆-alkyl, C₃-C₆-cycloalkyl, C₁-C₆-alkoxy, C₃-C₆-alkenyl or C₁-C₆-alkoxy-C₂-C₆-alkyl each of which is optionally mono- to trisubstituted by fluorine or chlorine, are phenyl or benzyl each of which is optionally mono- to trisubstituted by fluorine, chlorine, bromine, C₁-C₅-haloalkyl, C₁-C₅-alkyl or C₁-C₅-alkoxy, or together are a C₃-C₆-alkylene radical optionally substituted by C₁-C₄-alkyl in which optionally one methylene group is replaced by oxygen or sulphur.

3. Process for the preparation of compounds of the formula (I) according to Claim 1, in which
W is hydrogen, chlorine, bromine, methyl, ethyl, methoxy, ethoxy or trifluoromethyl,
X is hydrogen, chlorine, bromine, iodine, methyl, ethyl, propyl, cyclopropyl, methoxy, ethoxy, trifluoromethyl or trifluoromethoxy,
Y and Z are, independently of one another, hydrogen, fluorine, chlorine, bromine, iodine, methyl, ethyl, cyclopropyl, methoxy, trifluoromethyl, trifluoromethoxy or a phenyl radical, 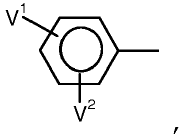
where in the case of phenyl, only one of the radicals Y or Z may be phenyl,
V¹ is hydrogen, fluorine or chlorine,
V² is hydrogen, fluorine, chlorine, methyl, ethyl, n-propyl, isopropyl, methoxy, ethoxy or trifluoromethyl,
A is hydrogen, C₁-C₄-alkyl or C₁-C₂-alkoxy-C₁-C₂-alkyl each of which is optionally mono- to trisubstituted by fluorine, is cyclopropyl, cyclopentyl or cyclohexyl,
B is hydrogen, methyl or ethyl or
A, B and the carbon atom to which they are bonded are saturated C₅-C₆-cycloalkyl in which optionally one ring member is replaced by nitrogen, oxygen or sulphur and which is optionally mono- or disubstituted by fluorine, chlorine, methyl, ethyl, methoxymethyl, ethoxymethyl, methoxyethyl, ethoxyethyl, trifluoromethyl, methoxy, ethoxy, propoxy, butoxy, methoxyethoxy, ethoxyethoxy, allyloxy, trifluoroethoxy or cyclopropylmethoxy, where the aforementioned radicals (with the exception of fluorine, chlorine and trifluoromethyl) are also suitable as N substituents,
A, B and the carbon atom to which they are bonded are C₆-cycloalkyl which is optionally substituted by an alkylidenediyl group optionally interrupted by a with a oxygen atom, or by an alkylidenediyl group containing with two non-directly adjacent oxygen atoms, where a 5- or 6-ring ketal is formed and which may in each case be optionally mono- or disubstituted by methyl, or
A, B and the carbon atom to which they are bonded are C₅-C₆-cycloalkyl or C₅-C₆-cycloalkenyl, in which two substituents, together with the carbon atoms to which they are bonded, are C₂-C₄-alkanediyl or C₂-C₄-alkenediyl or butadienediyl,
G is hydrogen (a) or one of the groups 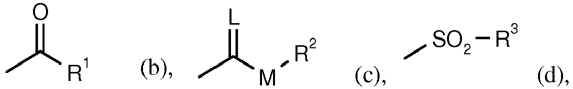 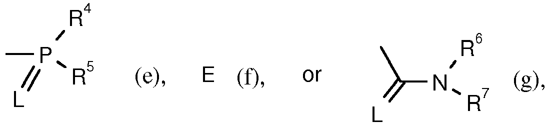
in which
E is a metal ion or an ammonium ion,
L is oxygen or sulphur and
M is oxygen or sulphur,
R¹ is C₁-C₁₀-alkyl, C₂-C₁₀-alkenyl, C₁-C₄-alkoxy-C₁-C₂-alkyl, C₁-C₄-alkylthio-C₁-C₂-alkyl each of which is optionally mono- to trisubstituted by fluorine or chlorine, or is C₃-C₆-cycloalkyl optionally monosubstituted by fluorine, chlorine, methyl, ethyl or methoxy,
is phenyl optionally mono- to disubstituted by fluorine, chlorine, bromine, cyano, nitro, methyl, ethyl, n-propyl, isopropyl, methoxy, ethoxy, trifluoromethyl or trifluoromethoxy,
is furanyl, thienyl or pyridyl each of which is optionally monosubstituted by chlorine, bromine or methyl,
R² is C₁-C₁₀-alkyl, C₂-C₁₀-alkenyl or C₁-C₄-alkoxy-C₂-C₄-alkyl each of which is optionally mono- to trisubstituted by fluorine or chlorine,
is cyclopentyl or cyclohexyl
or is phenyl or benzyl each of which is optionally mono- to disubstituted by fluorine, chlorine, cyano, nitro, methyl, ethyl, methoxy, trifluoromethyl or trifluoromethoxy,
R³ is methyl, ethyl, propyl or isopropyl each of which is optionally mono- to trisubstituted by fluorine or chlorine, or phenyl in each case optionally monosubtituted by fluorine, chlorine, bromine, methyl, ethyl, isopropyl, tert-butyl, methoxy, ethoxy, isopropoxy, trifluoromethyl, trifluoromethoxy, cyano or nitro,
R⁴ and R⁵, independently of one another, are C₁-C₄-alkoxy or C₁-C₄-alkylthio or are phenyl, phenoxy or phenylthio each of which is optionally monosubstituted by fluorine, chlorine, bromine, nitro, cyano, methyl, methoxy, trifluoromethyl or trifluoromethoxy,
R⁶ and R⁷, independently of one another, are hydrogen, C₁-C₄-alkyl, C₁-C₆-cycloalkyl, C₁-C₄-alkoxy, C₃-C₄-alkenyl or C₁-C₄-alkoxy-C₂-C₄-alkyl, phenyl optionally mono- to disubstituted by fluorine, chlorine, bromine, methyl, methoxy or trifluoromethyl, or together are a C₅-C₆-alkylene radical in which optionally one methylene group is replaced by oxygen or sulphur.

4. Process for the preparation of compounds of the formula (I) according to Claim 1, in which
W is hydrogen, chlorine, methyl, ethyl or methoxy,
X is hydrogen, chlorine, methyl, ethyl, methoxy or ethoxy,
Y and Z are, independently of one another, hydrogen, chlorine, methyl or the radical 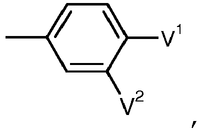
where in this case only one of the radicals Y or Z may be 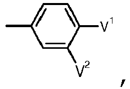
V¹ is hydrogen, fluorine or chlorine,
V² is hydrogen, fluorine or chlorine,
A is methyl, ethyl, propyl, isopropyl or cyclopropyl,
B is hydrogen or methyl,
A, B and the carbon atom to which they are bonded are saturated C₅-C₆-cycloalkyl in which optionally one ring member is replaced by oxygen and which is optionally monosubstituted by fluorine, chlorine, methyl, ethyl, methoxymethyl, methoxy, ethoxy, propoxy, butoxy, trifluoroethoxy, or
A, B and the carbon atom to which they are bonded are C₆-cycloalkyl which is optionally substituted by an alkylidenediyl group optionally interrupted by a with oxygen, or by an alkylidenediyl group containing with two non-directly adjacent oxygen atoms, where a 5- or 6-ring ketal is formed, each of which may be mono- or disubstituted by methyl,
G is hydrogen (a) or one of the groups 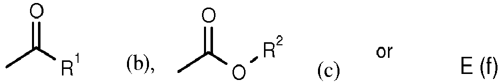
in which
E is a lithium, sodium, potassium, rubidium, caesium, magnesium, calcium ion or an ammonium ion,
R¹ is methyl, ethyl, propyl, isopropyl, butyl, isobutyl, sec-butyl, tert-butyl, cyclopropyl, cyclopentyl or cyclohexyl,
R² is methyl, ethyl, propyl, isopropyl, butyl, isobutyl, sec-butyl, tert-butyl, or is benzyl.

## Revendications

1. Procédé de fabrication de composés de formule (I) 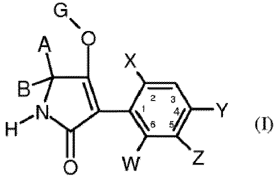 dans laquelle
W représente hydrogène, halogène, alkyle, alcényle, alcynyle, cycloalkyle éventuellement substitué, alcoxy, halogénoalkyle ou halogénoalcoxy,
X représente hydrogène, halogène, alkyle, alcényle, alcynyle, cycloalkyle éventuellement substitué, alcoxy, halogénoalkyle, halogénoalcoxy ou cyano,
Y et Z représentent indépendamment l'un de l'autre hydrogène, alkyle, alcényle, alcynyle, halogène, cyano, cycloalkyle éventuellement substitué, alcoxy, halogénoalkyle, halogénoalcoxy ; ou aryle ou hétaryle chacun éventuellement substitués,
A représente hydrogène ; alkyle, alcényle, alcoxyalkyle, alkylthioalkyle, chacun éventuellement substitués par halogène ; cycloalkyle saturé ou insaturé, éventuellement substitué, dans lequel au moins un atome de cycle est éventuellement remplacé par un hétéroatome ; ou aryle, arylalkyle ou hétaryle, chacun éventuellement substitués par halogène, alkyle, halogénoalkyle, alcoxy, halogénoalcoxy, cyano ou nitro,
B représente hydrogène, alkyle ou alcoxyalkyle, ou
A et B représentent ensemble avec l'atome de carbone auquel ils sont reliés un cycle saturé ou insaturé, contenant éventuellement au moins un hétéroatome, non substitué ou substitué,
G représente l'hydrogène (a) ou un des groupes 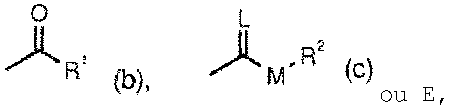 dans lesquels
E représente un ion métallique ou un ion ammonium,
L représente oxygène ou soufre,
M représente oxygène ou soufre,
R¹ représente alkyle, alcényle, alcoxyalkyle, alkylthioalkyle ou polyalcoxyalkyle, chacun éventuellement substitués par halogène ou cyano ; ou cycloalkyle ou hétérocyclyle, chacun éventuellement substitués par halogène, alkyle ou alcoxy ; ou phényle, phénylalkyle, hétaryle, phénoxyalkyle ou hétaryloxyalkyle, chacun éventuellement substitués,
R² représente alkyle, alcényle, alcoxyalkyle ou polyalcoxyalkyle, chacun éventuellement substitués par halogène ou cyano ; ou cycloalkyle, phényle ou benzyle, chacun éventuellement substitués,
**caractérisé en ce que** des composés de formule (X) 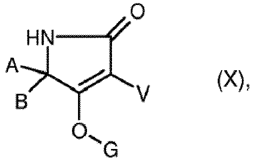 dans laquelle
A et B ont les significations indiquées précédemment,
G représente les groupes a), b), c) et E indiqués précédemment,
V représente l'hydrogène, ou
V représente COOR⁸,
R⁸ représentant alkyle,
sont mis en réaction avec un composé de formule (XI) 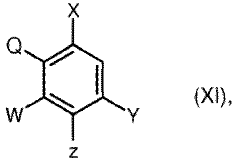 dans laquelle
W, X, Y et Z ont les significations indiquées précédemment, à l'exception que seuls fluor et chlore peuvent être représentés en tant qu'halogène, X peut également en outre représenter l'hydrogène, et
Q représente triflate, brome ou iode,
en présence d'une base, d'un catalyseur de palladium et d'un ligand phosphine de formule (XII') 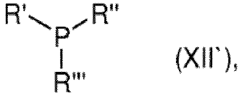 dans laquelle les radicaux
R', R" et R''' représentent indépendamment les uns des autres alkyle en C₁-C₁₂, cycloalkyle en C₅-C₁₀, aryle en C₆-C₁₀, éventuellement substitués une ou plusieurs fois par alkyle en C₁-C₆, alcoxy en C₁-C₆, alkylamino en C₁-C₆, dialkylamino en C₁-C₆; ou phényle éventuellement substitué une ou plusieurs fois par alkyle en C₁-C₆, alcoxy en C₁-C₆, alkylamino en C₁-C₆ ou dialkylamino en C₁-C₆,
dans un diluant.

2. Procédé de fabrication de composés de formule (I) selon la revendication 1, dans lequel
W représente hydrogène, chlore, brome, alkyle en C₁-C₄, alcényle en C₂-C₄, alcynyle en C₂-C₄; cycloalkyle en C₃-C₆ éventuellement substitué une fois par méthyle, éthyle, méthoxy, fluor, chlore, trifluorométhyle ou cyclopropyle ; alcoxy en C₁-C₄, halogénoalkyle en C₁-C₂ ou halogénoalcoxy en C₁-C₂,
X représente hydrogène, chlore, brome, iode, alkyle en C₁-C₄, alcényle en C₂-C₄, alcynyle en C₂-C₄; cycloalkyle en C₃-C₆ éventuellement substitué une fois par méthyle, éthyle, méthoxy, fluor, chlore, trifluorométhyle ou cyclopropyle ; alcoxy en C₁-C₄, halogénoalkyle en C₁-C₄, halogénoalcoxy en C₁-C₄ ou cyano,
Y et Z représentent indépendamment l'un de l'autre hydrogène, cyano, fluor, chlore, brome, iode, alkyle en C₁-C₄, alcényle en C₂-C₄, alcynyle en C₂-C₄ ; cycloalkyle en C₃-C₆ éventuellement substitué une fois par méthyle, éthyle, méthoxy, fluor, chlore, trifluorométhyle ou cyclopropyle ; alcoxy en C₁-C₆, halogénoalkyle en C₁-C₄, halogénoalcoxy en C₁-C₄ ou un des radicaux (hét)-aryle, 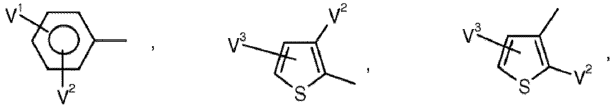 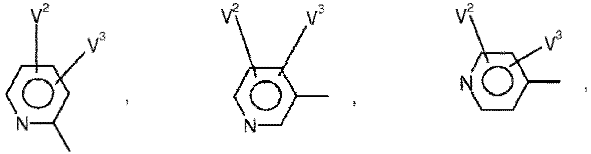 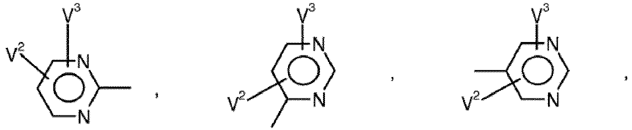 dans le cas d' (hét) -aryle, seul un des radicaux Y ou Z pouvant représenter (hét)-aryle,
V¹ représente hydrogène, fluor, chlore, brome, alkyle en C₁-C₆, alcoxy en C₁-C₄, halogénoalkyle en C₁-C₂, halogénoalcoxy en C₁-C₂, nitro, cyano ; ou phényle éventuellement substitué une fois à deux fois par fluor, chlore, brome, alkyle en C₁-C₄, alcoxy en C₁-C₄, halogénoalkyle en C₁-C₂, halogénoalcoxy en C₁-C₂, nitro ou cyano,
V² et V³ représentent indépendamment l'un de l'autre hydrogène, fluor, chlore, brome, alkyle en C₁-C₄, alcoxy en C₁-C₄, halogénoalkyle en C₁-C₂ ou halogénoalcoxy en C₁-C₂,
A représente hydrogène ; alkyle en C₁-C₆, alcoxy en C₁-C₄-alkyle en C₁-C₂, chacun éventuellement substitués une fois à trois fois par fluor ou chlore ; cycloalkyle en C₃-C₆ éventuellement substitué une fois à deux fois par alkyle en C₁-C₂ ou alcoxy en C₁-C₂ et éventuellement interrompu par un atome d'oxygène ; ou phényle, pyridyle ou benzyle, chacun éventuellement substitués une fois à deux fois par fluor, chlore, brome, alkyle en C₁-C₄, halogénoalkyle en C₁-C₂, alcoxy en C₁-C₄, halogénoalcoxy en C₁-C₂, cyano ou nitro,
B représente hydrogène, alkyle en C₁-C₄ ou alcoxy en C₁-C₂-alkyle en C₁-C₂, ou
A, B et l'atome de carbone auquel ils sont reliés représentent un cycloalkyle en C₃-C₇ saturé ou insaturé, dans lequel un élément de cycle est éventuellement remplacé par azote, oxygène ou soufre, et qui est éventuellement substitué une fois à deux fois par halogène, alkyle en C₁-C₆, alcoxy en C₁-C₄-alkyle en C₁-C₂, trifluorométhyle, alcoxy en C₁-C₆, alcényloxy en C₃-C₆, trifluoroéthoxy, alcoxy en C₁-C₃-alcoxy en C₁-C₃ ou cycloalkylméthoxy en C₃-C₆, les radicaux mentionnés précédemment (à l'exception d'halogène et de trifluorométhyle) pouvant également être envisagés en tant que N-substituants, ou
A, B et l'atome de carbone auquel ils sont reliés représentent cycloalkyle en C₅-C₆, qui est substitué par un groupe alkylènediyle contenant éventuellement un ou deux atomes d'oxygène ou de soufre non directement voisins, éventuellement substitué par méthyle ou éthyle, ou par un groupe alkylènedioxyle ou par un groupe alkylènedithiol, qui forme avec l'atome de carbone auquel il est relié un cycle à cinq ou six chaînons supplémentaire, ou
A, B et l'atome de carbone auquel ils sont reliés représentent cycloalkyle en C₃-C₆ ou cycloalcényle en C₅-C₆, dans lesquels deux substituants représentent ensemble avec les atomes de carbone auxquels ils sont reliés alcanediyle en C₂-C₄, alcènediyle en C₂-C₄ ou butadiènediyle, chacun éventuellement substitués par alkyle en C₁-C₂ ou alcoxy en C₁-C₂,
G représente l'hydrogène (a) ou un des groupes 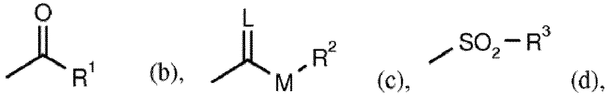 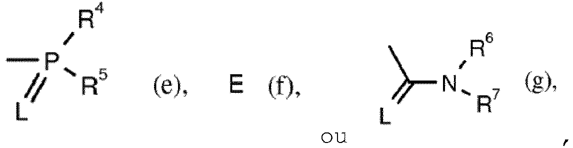 dans lesquels
E représente un ion métallique ou un ion ammonium,
L représente oxygène ou soufre, et
M représente oxygène ou soufre,
R¹ représente alkyle en C₁-C₁₆, alcényle en C₂-C₁₆, alcoxy en C₁-C₆-alkyle en C₁-C₄, alkylthio en C₁-C₆-alkyle en C₁-C₄ ou poly-alcoxy en C₁-C₆-alkyle en C₁-C₄, chacun éventuellement substitués une fois à trois fois par fluor ou chlore ; ou cycloalkyle en C₃-C₇ éventuellement substitué une fois à deux fois par fluor, chlore, alkyle en C₁-C₅ ou alcoxy en C₁-C₅, dans lequel un ou deux groupes méthylène non directement voisins sont éventuellement remplacés par oxygène et/ou soufre,
phényle éventuellement substitué une fois à trois fois par fluor, chlore, brome, cyano, nitro, alkyle en C₁-C₄, alcoxy en C₁-C₄, halogénoalkyle en C₁-C₃, halogénoalcoxy en C₁-C₃, alkylthio en C₁-C₄ ou alkylsulfonyle en C₁-C₄,
phényl-alkyle en C₁-C₄ éventuellement substitué une fois à deux fois par fluor, chlore, brome, alkyle en C₁-C₄, alcoxy en C₁-C₄, halogénoalkyle en C₁-C₃ ou halogénoalcoxy en C₁-C₃,
pyrazolyle, thiazolyle, pyridyle, pyrimidyle, furanyle ou thiényle, chacun éventuellement substitués une fois à deux fois par fluor, chlore, brome ou alkyle en C₁-C₄,
phénoxy-alkyle en C₁-C₅ éventuellement substitué une fois à deux fois par fluor, chlore, brome ou alkyle en C₁-C₄, ou
pyridyloxy-alkyle en C₁-C₅, pyrimidyloxy-alkyle en C₁-C₅ ou thiazolyloxy-alkyle en C₁-C₅, chacun éventuellement substitués une fois à deux fois par fluor, chlore, brome, amino ou alkyle en C₁-C₄,
R² représente alkyle en C₁-C₁₆, alcényle en C₂-C₁₆, alcoxy en C₁-C₆-alkyle en C₂-C₆ ou poly-alcoxy en C₁-C₆-alkyle en C₂-C₆, chacun éventuellement substitués une fois à trois fois par fluor ou chlore,
cycloalkyle en C₃-C₇ éventuellement substitué une fois à deux fois par fluor, chlore, alkyle en C₁-C₄ ou alcoxy en C₁-C₄, ou
phényle ou benzyle, chacun éventuellement substitués une fois à trois fois par fluor, chlore, brome, cyano, nitro, alkyle en C₁-C₄, alcoxy en C₁-C₃, halogénoalkyle en C₁-C₃ ou halogénoalcoxy en C₁-C₃,
R³ représente alkyle en C₁-C₆ éventuellement substitué une fois à trois fois par fluor ou chlore ; ou phényle ou benzyle chacun éventuellement substitués une fois à deux fois par fluor, chlore, brome, alkyle en C₁-C₄, alcoxy en C₁-C₄, halogénoalcoxy en C₁-C₂, halogénoalkyle en C₁-C₂, cyano ou nitro,
R⁴ et R⁵ représentent indépendamment l'un de l'autre alkyle en C₁-C₆, alcoxy en C₁-C₆, alkylamino en C₁-C₆, di-(alkyle en C₁-C₆) -amino, alkylthio en C₁-C₆ ou alcénylthio en C₃-C₄, chacun éventuellement substitués une fois à trois fois par fluor ou chlore ; ou phényle, phénoxy ou phénylthio, chacun éventuellement substitués une fois à deux fois par fluor, chlore, brome, nitro, cyano, alcoxy en C₁-C₃, halogénoalcoxy en C₁-C₃, alkylthio en C₁-C₃, halogénoalkylthio en C₁-C₃, alkyle en C₁-C₃ ou halogénoalkyle en C₁-C₃,
R⁶ et R⁷ représentent indépendamment l'un de l'autre hydrogène ; alkyle en C₁-C₆, cycloalkyle en C₃-C₆, alcoxy en C₁-C₆, alcényle en C₃-C₆ ou alcoxy en C₁-C₆-alkyle en C₂-C₆, chacun éventuellement substitués une fois à trois fois par fluor ou chlore ; phényle ou benzyle, chacun éventuellement substitués une fois à trois fois par fluor, chlore, brome, halogénoalkyle en C₁-C₅, alkyle en C₁-C₅ ou alcoxy en C₁-C₅ ; ou représentent ensemble un radical alkylène en C₃-C₆ éventuellement substitué par alkyle en C₁-C₄, dans lequel un groupe méthylène est éventuellement remplacé par oxygène ou soufre.

3. Procédé de fabrication de composés de formule (I) selon la revendication 1, dans lequel
W représente hydrogène, chlore, brome, méthyle, éthyle, méthoxy, éthoxy ou trifluorométhyle,
X représente hydrogène, chlore, brome, iode, méthyle, éthyle, propyle, cyclopropyle, méthoxy, éthoxy, trifluorométhyle ou trifluorométhoxy,
Y et Z représentent indépendamment l'un de l'autre hydrogène, fluor, chlore, brome, iode, méthyle, éthyle, cyclopropyle, méthoxy, trifluorométhyle, trifluorométhoxy ou un radical phényle, 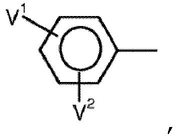 dans le cas de phényle, seul un des radicaux Y ou Z pouvant représenter phényle,
V¹ représente hydrogène, fluor ou chlore,
V² représente hydrogène, fluor, chlore, méthyle, éthyle, n-propyle, iso-propyle, méthoxy, éthoxy ou trifluorométhyle,
A représente hydrogène ; alkyle en C₁-C₄ ou alcoxy en C₁-C₂-alkyle en C₁-C₂, chacun éventuellement substitués une fois à trois fois par fluor ; cyclopropyle, cyclopentyle ou cyclohexyle,
B représente hydrogène, méthyle ou éthyle, ou
A, B et l'atome de carbone auquel ils sont reliés représentent un cycloalkyle en C₅-C₆ saturé, dans lequel un élément de cycle est éventuellement remplacé par azote, oxygène ou soufre, et qui est éventuellement substitué une fois ou deux fois par fluor, chlore, méthyle, éthyle, méthoxyméthyle, éthoxyméthyle, méthoxyéthyle, éthoxyéthyle, trifluorométhyle, méthoxy, éthoxy, propoxy, butoxy, méthoxyéthoxy, éthoxyéthoxy, allyloxy, trifluoroéthoxy ou cyclopropylméthoxy, les radicaux mentionnés précédemment (à l'exception de fluor, chlore, trifluorométhyle) pouvant également être envisagés en tant que N-substituants,
A, B et l'atome de carbone auquel ils sont reliés représentent cycloalkyle en C₆, qui est éventuellement substitué par un groupe alkylidènediyle éventuellement interrompu par un atome d'oxygène, ou par un groupe alkylidènediyle contenant deux atomes d'oxygène non directement voisins, un cétal cyclique en 5 ou 6 étant formé, et qui peuvent chacun éventuellement être substitués une fois ou deux fois par méthyle, ou
A, B et l'atome de carbone auquel ils sont reliés représentent cycloalkyle en C₅-C₆ ou cycloalcényle en C₅-C₆, dans lesquels deux substituants représentent ensemble avec les atomes de carbone auxquels ils sont reliés alcanediyle en C₂-C₄ ou alcènediyle en C₂-C₄ ou butadiènediyle,
G représente l'hydrogène (a) ou un des groupes 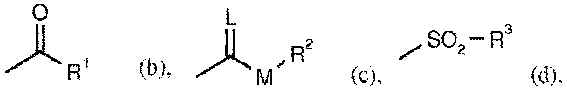 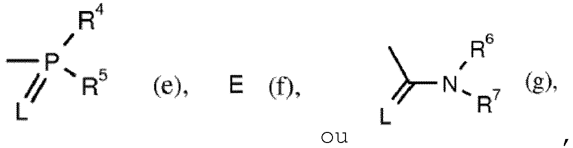 dans lesquels
E représente un ion métallique ou un ion ammonium,
L représente oxygène ou soufre, et
M représente oxygène ou soufre,
R¹ représente alkyle en C₁-C₁₀, alcényle en C₂-C₁₀, alcoxy en C₁-C₄-alkyle en C₁-C₂, alkylthio en C₁-C₄-alkyle en C₁-C₂, chacun éventuellement substitués une fois à trois fois par fluor ou chlore ; ou cycloalkyle en C₃-C₆ éventuellement substitué une fois par fluor, chlore, méthyle, éthyle ou méthoxy,
phényle éventuellement substitué une fois à deux fois par fluor, chlore, brome, cyano, nitro, méthyle, éthyle, n-propyle, méthoxy, éthoxy, trifluorométhyle ou trifluorométhoxy,
furanyle, thiényle ou pyridyle, chacun éventuellement substitués une fois par chlore, brome ou méthyle,
R² représente alkyle en C₁-C₁₀, alcényle en C₂-C₁₀ ou alcoxy en C₁-C₄-alkyle en C₂-C₄, chacun éventuellement substitués une fois à trois fois par fluor ou chlore,
cyclopentyle ou cyclohexyle,
ou phényle ou benzyle, chacun éventuellement substitués une fois à deux fois par fluor, chlore, cyano, nitro, méthyle, éthyle, méthoxy, trifluorométhyle ou trifluorométhoxy,
R³ représente méthyle, éthyle, propyle ou iso-propyle, chacun éventuellement substitués une fois à trois fois par fluor ou chlore ; ou phényle éventuellement substitué une fois par fluor, chlore, brome, méthyle, éthyle, iso-propyle, tert.-butyle, méthoxy, éthoxy, iso-propoxy, trifluorométhyle, trifluorométhoxy, cyano ou nitro,
R⁴ et R⁵ représentent indépendamment l'un de l'autre alcoxy en C₁-C₄ ou alkylthio en C₁-C₄; ou phényle, phénoxy ou phénylthio, chacun éventuellement substitués une fois par fluor, chlore, brome, nitro, cyano, méthyle, méthoxy, trifluorométhyle ou trifluorométhoxy, R⁶ et R⁷ représentent indépendamment l'un de l'autre hydrogène ; alkyle en C₁-C₄, cycloalkyle en C₃-C₆, alcoxy en C₁-C₄, alcényle en C₃-C₄ ou alcoxy en C₁-C₄-alkyle en C₂-C₄; phényle éventuellement substitué une fois à deux fois par fluor, chlore, brome, méthyle, méthoxy ou trifluorométhyle ; ou représentent ensemble un radical alkylène en C₅-C₆ dans lequel un groupe méthylène est éventuellement remplacé par oxygène ou soufre.

4. Procédé de fabrication de composés de formule (I) selon la revendication 1, dans lequel
W représente hydrogène, chlore, méthyle, éthyle ou méthoxy,
X représente hydrogène, chlore, méthyle, éthyle, méthoxy ou éthoxy,
Y et Z représentent indépendamment l'un de l'autre hydrogène, chlore, méthyle ou le radical 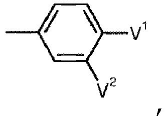 dans ce cas, seul un des radicaux Y ou Z pouvant représenter 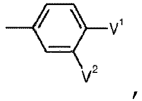 V¹ représente hydrogène, fluor ou chlore,
V² représente hydrogène, fluor ou chlore,
A représente méthyle, éthyle, propyle, iso-propyle ou cyclopropyle,
B représente hydrogène ou méthyle,
A, B et l'atome de carbone auquel ils sont reliés représentent un cycloalkyle en C₅-C₆ saturé, dans lequel un élément de cycle est éventuellement remplacé par oxygène, et qui est éventuellement substitué une fois par fluor, chlore, méthyle, éthyle, méthoxyméthyle, méthoxy, éthoxy, propoxy, butoxy, trifluoroéthoxy, ou
A, B et l'atome de carbone auquel ils sont reliés représentent cycloalkyle en C₆, qui est éventuellement substitué par un groupe alkylidènediyle éventuellement interrompu par un oxygène, ou par un groupe alkylidènediyle contenant deux atomes d'oxygène non directement voisins, un cétal cyclique en 5 ou 6 étant formé, qui peuvent chacun être substitués une fois ou deux fois par méthyle,
G représente l'hydrogène (a) ou un des groupes 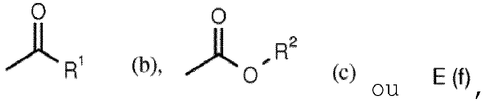 dans lesquels
E représente un ion lithium, sodium, potassium, rubidium, césium, magnésium, calcium ou un ion ammonium,
R¹ représente méthyle, éthyle, propyle, iso-propyle, butyle, iso-butyle, sec.-butyle, tert.-butyle, cyclopropyle, cyclopentyle ou cyclohexyle,
R² représente méthyle, éthyle, propyle, iso-propyle, butyle, iso-butyle, sec.-butyle, tert.-butyle ou benzyle.
